# EUROPEAN PATENT SPECIFICATION

(11) **EP 3 331 534 B1**
(45) Date of publication and mention of the grant of the patent: **18.12.2019**
(21) Application number: 16750736.7
(22) Date of filing: 04.08.2016
(51) Int. Cl.: A61K 31/7028, C07H 3/06, A23L 33/135

(54) **NUTRITIONAL COMPOSITIONS WITH 2FL AND LNNT FOR USE IN INDUCING A GUT MICROBIOTA CLOSE TO THE ONE OF BREAST FED INFANTS**
NÄHRSTOFFZUSAMMENSETZUNG MIT 2FL UND LNNT ZUM INDUZIEREN EINER DARMBIOZÖNOSE IN DER NÄHE GESTILLTER KLEINKINDER
COMPOSITIONS NUTRITIONNELLES AVEC 2FL ET LNNT POUR UTILISATION DANS L'INDUCTION D'UNE FLORE INTESTINALE PROCHE D'UN NOURRISSON ALLAITÉ

(30) Priority: 04.08.2015 EP 15179742
(43) Date of publication of application: 13.06.2018
(73) Proprietor: Société des Produits Nestlé S.A., 1800 Vevey (CH)
(72) Inventor: BERGER, Bernard, 1613 Maracon (CH); SPRENGER, Norbert, 1073 Savigny (CH)
(74) Representative: Corticchiato, Olivier
(86) International application number: PCT/EP2016/068597
(87) International publication number: WO 2017/021476

(56) References cited:
- EP-A1- 2 455 387
- EP-A1- 2 465 508
- WO-A1-2009/060073
- WO-A1-2012/158517
- WO-A1-2013/057049
- WO-A1-2015/071402
- US-A- 6 146 670

## Description

### Field of the invention

The present invention relates to nutritional compositions for infants or young children and their health effects. In particular, it relates to infant formula comprising specific oligosaccharides for inducing a gut microbiota that is close to the one of infants fed exclusively human breast milk (HBM).

### Background of the invention

Mother's milk is recommended for all infants. However, in some cases breast feeding is inadequate or unsuccessful for medical reasons or the mother chooses not to breast feed. Nutritional compositions such as infant formulae have been developed for these situations.

Nutritional compositions for infants and young children are often sold as powders to be reconstituted with water or in some instances as ready to drink or concentrated liquid compositions. Those compositions are intended to cover most or all the nutritional needs of the infants or young children.

It is known however, that human breast milk represents the ultimate gold standard in terms of infants' nutrition. Infant formula manufacturers have therefore made many attempts to induce nutritional health effects close to or similar to the benefits of human breast milk. However many studies have shown that infant formula do not induce the identical effects on the body compared to human breast milk. For example, infants fed infant formula and infants fed human-breast milk (HBM) can exhibit a different intestinal (gut) microbiota.

Infancy, especially the first weeks, 3 months, 6 months or 12 months of life is a critical period for the establishment of a balanced gut microbiota.

It is know that the modulation of the gut microbiota during infancy can prospectively have a great influence in the future health status of the bodies. For example the gut flora can have influence on the development of a strong immune system later in life, a normal growth and even on the development of obesity later in life.

Similarly, a healthy intestinal flora is an indicator of the health of an infant and an altered intestinal microbiota can be an indicator (and/or a cause) of abnormal health events such as diarrhea, under-absorption of nutrients, colic, altered sleep and/or altered growth and development.

It is known that the mode of delivery can also affect the initial gut microbiota of infants: infants delivered by Caesarean section (C-section) have been shown to have a different gut microbiota compared to vaginally-delivered infants.

The gut microbiota and its evolution during the development of the infant is, however, a fine balance between the presence and prevalence (amount) of many populations of gut bacteria. Some gut bacteria are classified as "generally positive" while other ones are "generally negative" (or pathogenic) as to their effect on the overall health of the infant.

Certain species of "generally positive" bacteria, such as bifidobacteria, may be underrepresented in infants fed conventional infant formula in comparison to breast fed infants. Similarly some bacterial populations are considered pathogenic and should remain of low prevalence in the gut microbiota.

Indeed infant fed infant formulae may not benefit from the natural, well balanced intestinal gut flora (gut microbiota) of infants fed exclusively or predominantly Human Breast Milk. Such natural microbiota observed in breast fed infants is indeed both very well controlled over time (evolution over time) and complex. Many taxa of microorganisms co-exist in the highly complex microenvironment of the gut/intestine, each in sequentially defined proportions. Quantitative and qualitative dimensions are to be considered when defining the microbiota of infants or young children. Furthermore, the variation over time of the gut microbiota adds to the complexity.

A suitable and healthy gut microbiota is a key factor in the development of the mucosal immune system of the infant. While many studies have identified ways to promote the growth and prevalence of specific positive bacteria in the gut of infants, little is known about ways to induce a microbiota that resemble the one of breast-fed infants.

The use of probiotics has especially been investigated. Probiotics are considered to be viable microbial preparations which promote the individual's health by preserving the natural microflora in the intestine. Probiotics are deemed to attach to the intestine's mucosa, colonize the intestinal tract and likewise prevent attachment of harmful microorganisms thereon. A crucial prerequisite for their action resides in that they have to reach the gut's mucosa in a proper and viable form and do not get destroyed in the upper part of the gastrointestinal tract, especially by the influence of the low pH prevailing in the stomach. Another difficulty is that gut microbiota is very diversified and complex, and bacteria have various interactions in-between.

It is known that, amongst other ingredients, non-digestible carbohydrates (prebiotics) in particular can affect the promotion of particular microbiota. For example, it has been shown that certain galacto-oligosaccharides (GOS) and/or certain fructo-oligosaccharides (FOS) can promote the growth and prevalence of bifidobacteria in the gut, especially in infants.

In particular, human milk contains an abundance of structurally diverse oligosaccharides, known collectively as human milk oligosaccharides (HMOs), which may support immune function through several probable mechanisms. These include a prebiotic effect resulting in the development and maintenance of a healthy gut microbiota, a key factor in the development of the mucosal immune system (Bode et al, Human milk oligosaccharides: Every baby needs a sugar mama. Glycobiology 2012;22(9):1147-1162). HMOs may also function as soluble decoy receptors in the gut, protecting the neonate from enteric pathogens (Newburg et al, Human milk glycans protect infants against enteric pathogens." Annual Review of Nutrition 2005;25:37-58) and may also directly interact with gut epithelial cells yielding changes that may interfere with host-microbial interactions (Bode et al, 2012).

WO9843495 from Abbott refers to a nutritional formulation containing an effective amount of Lacto-N-neoTetraose to simulate the growth and/or metabolic activity of Bifidobacterium infantis.

WO2009060073 from Nestec SA relates to the use of an oligosaccharide such as lacto-N-tetraose or lacto-N-neotetraose to promote the development in the first few weeks of the life of the infant of a beneficial intestinal microbiota comparable with that found in breast fed infants, especially an intestinal microbiota dominated by appreciable populations of Bifidobacterium and Lactobacillus species to the exclusion of other populations such as species Bacteroides, Clostridia and Streptococci. WO2012158517 discloses the use of purified HMOs like 2'-FL, 3-FL, or LDFT for stimulating the growth of bacteria in a gastrointestinal tract of a mammalian subject, including Bifidobacteria.

The existing interventions with probiotics and/or prebiotics however modulate specific microbiota taxa, e.g. they result in an increased number of bifidobacteria or a decreased number of clostridia. However, no solution is currently available to bring the global microbiota composition (i.e. the overall/entire/total/whole microbiota) of formula-fed infants closer to that of breast-fed infants.

No existing solutions seem to also take into account the gut microbial function.

There is therefore a need for infants fed with infant formula, to promote and/or induce an overall microbiota that is close to the microbiota of breast-fed infants, both in terms of composition and function.

There is a need for nutritional compositions for infants or young children that promote and/or induce over time a global microbiota that evolves in a similar manner as the one of breast-fed infants.

There is a need to provide infants or young children with the best nutrition that enables the development of a global microbiota close to the one of breast-fed infants, said development being short term (i.e. during the nutritional intervention) and/or long term (i.e. after the nutritional intervention).

There is a need for nutritional compositions for infants or young children that induce an optimal short term or long term health status through a nutrition inducing and/or promoting development of a global microbiota close/similar to the one of breast-fed infants; such health status including an optimum growth over time, and an optimum development of the immune system, as well as the prevention of metabolic disorders.

There is a need to compensate for the sub-normal overall microbiota observed in non-breast-fed infants or young children, e.g. those fed a conventional nutritional composition. There is a need to rebalance such overall microbiota.

There is a need to enhance a good balance in the overall gut microbiota of infants, especially by down-regulating or repressing the growth of pathogenic bacteria, during the first weeks of life when such a balance is being established.

There is a need for nutritional compositions for infants or young children that provide with a global microbiota and a metabolic signature closer to the ones obtained for breast-fed infants.

There is a need for nutritional compositions for infants or young children that provide a healthy growth, a healthy immune system, a healthy gut function and/or prevent microbiota dysbiosis in said infants or young children, e.g. immediately or later in life.

There is a need to deliver such health benefits in these infants or young children in a manner that does not induce side effects and/or in a manner that is easy to deliver, and well accepted by the parents or health care practitioners.

### Summary of the invention

The present inventors have found that a composition comprising at least one fucosylated oligosaccharide (2FL) and at least one N-acetylated oligosaccharide (LNnT) can advantageously be used to provide in infants a global microbiota in the gut that is closer to the one of infants fed exclusively with human breast milk, in comparison to the global microbiota in the gut of infants fed with a conventional infant formula not comprising said oligosaccharides. Together the stool microbiota and metabolic signature show that the addition of 2 individual and structurally very specific human milk oligosaccharides (HMOs), shifts the overall gut microbiota, evaluated in stools, both in terms of composition and function towards that observed in breast-fed infants. Without wishing to be bound by theory it is believed that these oligosaccharides act synergistically for getting such an impact on the global microbiota in the gut.

Accordingly, the present invention therefore refers to a nutritional composition comprising at least one fucosylated oligosaccharide and at least one N-acetylated oligosaccharide for use in promoting and/or inducing in infants or young children a global microbiota in the gut that is closer to the global microbiota in the gut of infants or young children fed exclusively with human breast milk, in comparison to the global microbiota in the gut of infants or young children fed predominantly or exclusively with a conventional nutritional composition not comprising said oligosaccharides.

The nutritional composition of the present invention has the advantage to provide an effect on the composition and/or the function of the entire microbiota in the gut, such as the relative taxonomic abundance (or amount), the diversity, the activity and/or the functionality of said microbiota.

It can be especially used in providing a healthy growth, in providing a healthy immune system, in providing a healthy gut function and/or in preventing microbiota dysbiosis in infants or young children.

In a particularly advantageous embodiment, the nutritional composition comprises 2'-fucosyllactose (2-FL) and lacto-N-neotetraose (LNnT), and especially 2'-fucosyllactose (2-FL) in an amount of 0.8-1.5 g/L of the nutritional composition and LNnT in an amount of 0.5-0.8 g/L of the nutritional composition.

### Figures

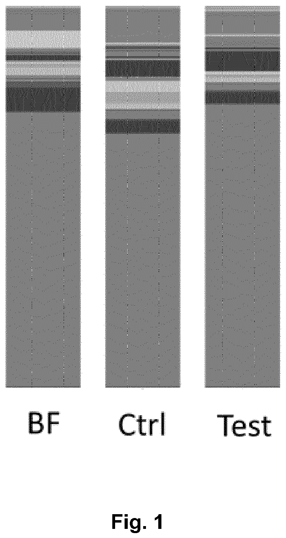

Figure 1 represents the general profile of the global average microbiota at genus level between breast-fed reference (BF), Control (Ctrl) and Test groups, as measured by 16S rRNA gene profiling.

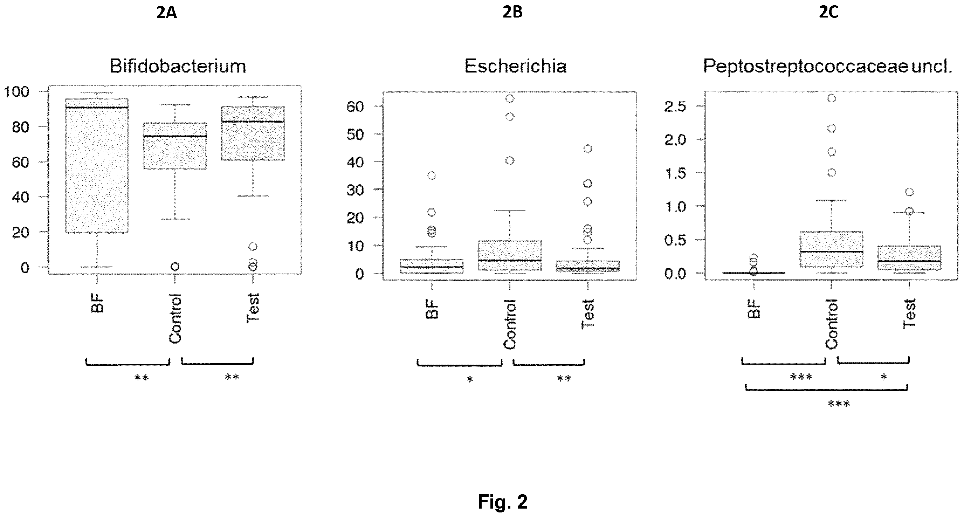

Figures 2 represent the relative composition of the three groups - BF, control and Test groups - for the three main taxa that showed significant differences between the Test and Control groups, as measured by 16S rRNA gene profiling: *Bifidobacterium* (figure 2A), *Escherichia* (figure 2B) and *Peptostreptococacceae uncl* (figure 2C). Median with interquartile ranges is depicted. Significant difference indicated by *, p<0.05; **, p<0.01; ***, p<0.001. BF, breast-fed reference group.

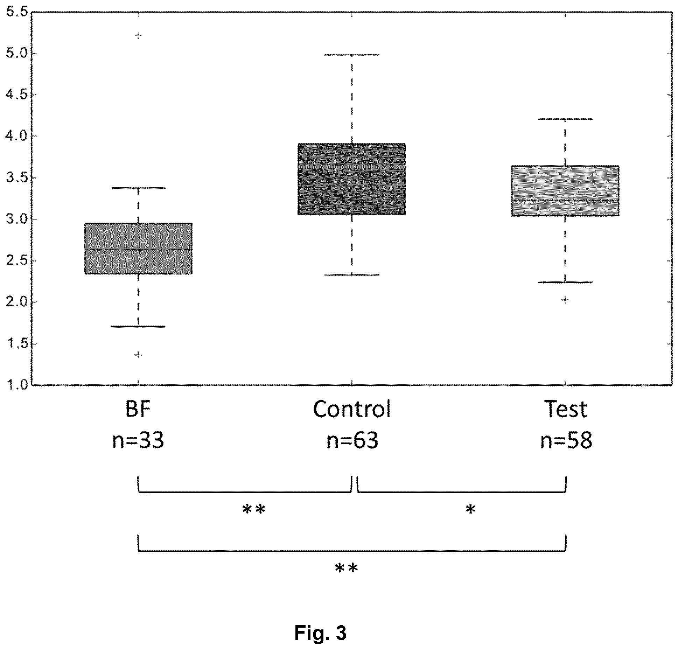

Figure 3 represents the alpha diversity of the global microbiota of the three groups - BF, control and Test groups - calculated with PD_whole_tree based on 16S rRNA profiling. Significant difference indicated by *, p<0.05; **, p<0.01

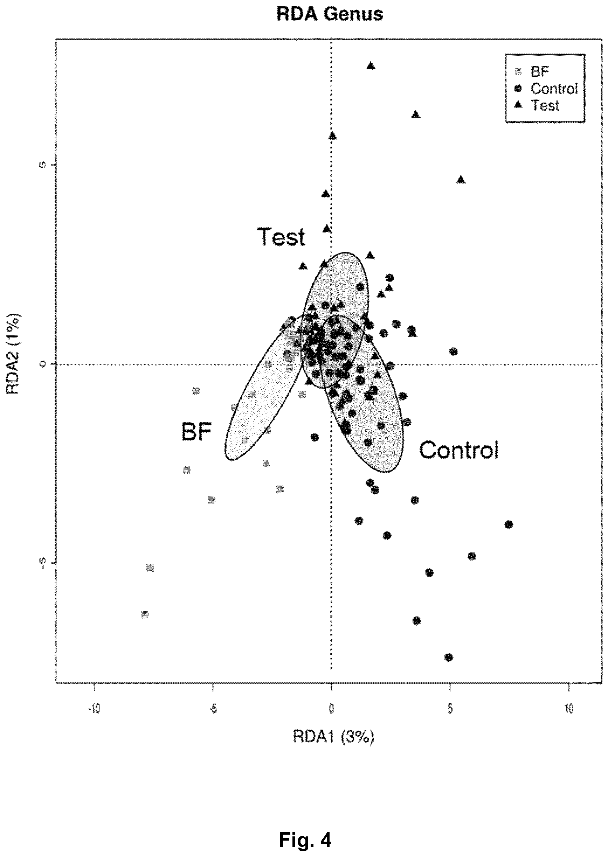

Figure 4 represents the redundancy analysis based on data at the genus level, as measured by 16S rRNA gene profiling, and shows a significant separation of the three groups. p<0.001.

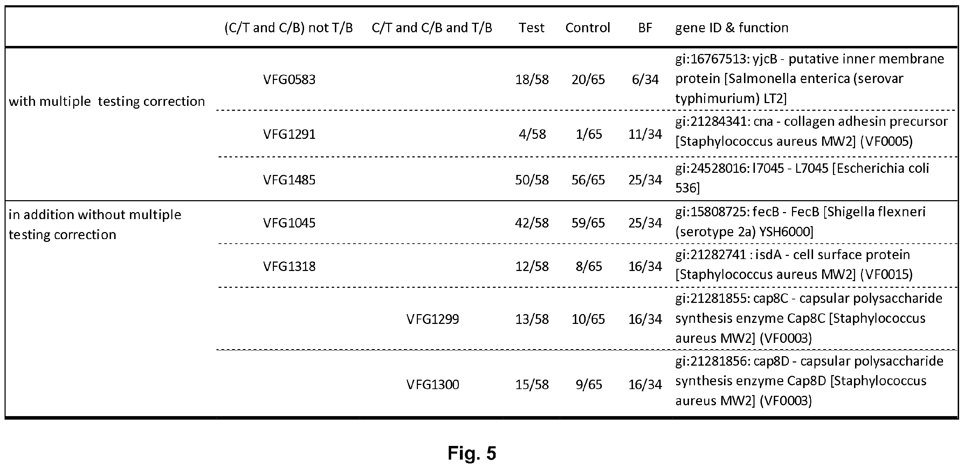

Figure 5 is a table showing the list of detected genes encoding known virulence factors, as measured by metagenomics. C/T, C/B and T/B stand for significant difference between Control and Test groups, Control and Breast-fed groups and Test and Breast-fed groups respectively. Significance between groups was assessed by fitting a negative binomial regression model accounting for excess zero-count data if needed. The number of infants with detected genes is also shown for each group.

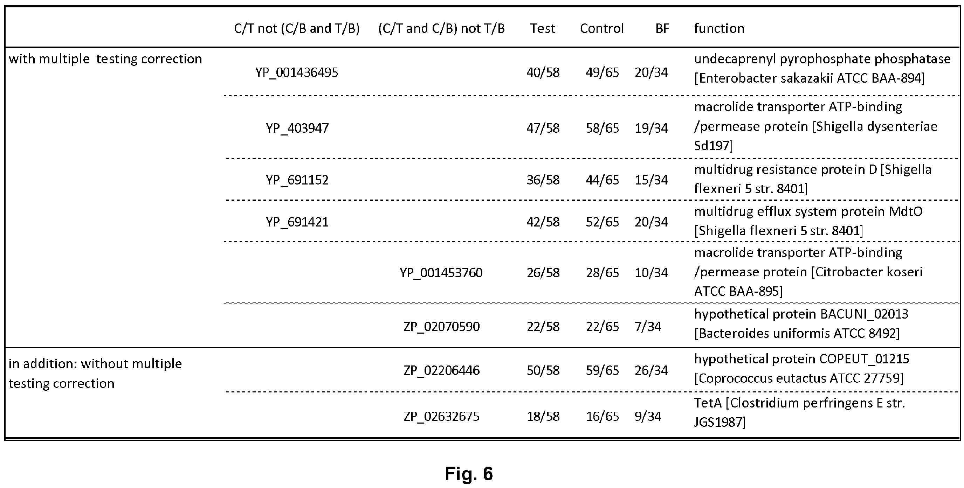

Figure 6 is a table showing the list of detected genes encoding known antibiotic resistance genes, as measured by metagenomics. C/T, C/B and T/B stand for significant difference between Control and Test groups, Control and Breast-fed groups and Test and Breast-fed groups respectively. Significance between groups was assessed by fitting a negative binomial regression model accounting for excess zero-count data if needed. The number of infants with detected genes is also shown for each group.

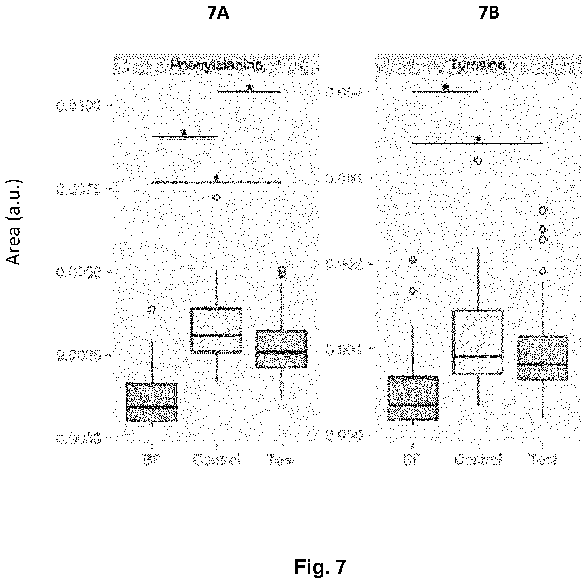

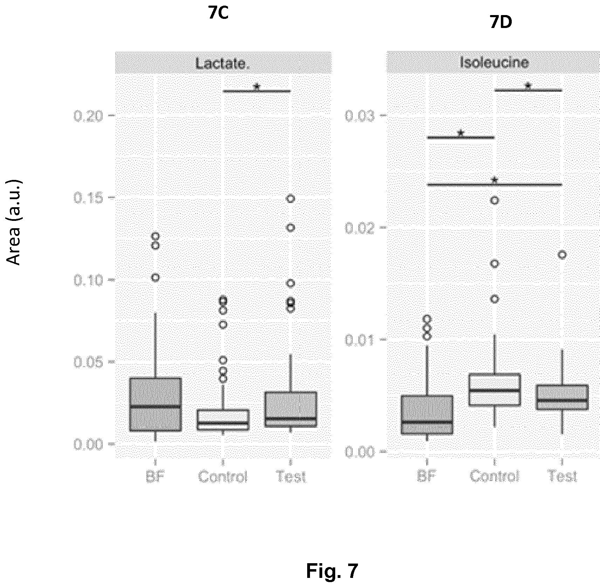

Figures 7 represent Relative concentration of Influential metabolites derived from 1H NMR spectroscopic stool data related to amino acid and other organic acid metabolism: phenylalanine (figure 7A), tyrosine (figure 7B), lactate (figure 7C) and isoleucine (figure 7D). * Indicate significant difference by Kruskal-Wallis (p<0.05). BF, breast-fed reference group.

### Detailed description of the invention

As used herein, the following terms have the following meanings.

The term "**infant**" means a child under the age of 12 months.

The expression "**young child**" means a child aged between one and three years, also called toddler.

An **"infant or young child born by C-section"** means an infant or young child who was delivered by caesarean. It means that the infant or young child was not vaginally delivered.

An **"infant or young child vaginally born"** means an infant or young child who was vaginally delivered and not delivered by caesarean.

A **"preterm"** or **"premature"** means an infant or young child who was not born at term. Generally it refers to an infant or young child born prior to the completion of 37 weeks of gestation.

By the expression **"small for gestational age"** or **"SGA",** it is intended to mean an infant or young child who is smaller in size than normal for their gestational age at birth, most commonly defined as a weight below the 10th percentile for the gestational age. In some embodiments, SGA may be associated with Intrauterine growth restriction (IUGR), which refers to a condition in which a foetus is unable to achieve its potential size.

By the expression **"low birth weight",** it should be understood as any body weight under 2500g at birth.

The expression **"nutritional composition"** means a composition which nourishes a subject. This nutritional composition is usually to be taken orally or intravenously, and it usually includes a lipid or fat source and a protein source.

In a particular embodiment the nutritional composition of the present invention is a hypoallergenic nutritional composition. The expression **"hypoallergenic nutritional composition"** means a nutritional composition which is unlikely to cause allergic reactions.

In a particular embodiment the nutritional composition of the present invention is a "synthetic nutritional composition". The expression **"synthetic nutritional composition"** means a mixture obtained by chemical and/or biological means, which can be chemically identical to the mixture naturally occurring in mammalian milks (i.e. the synthetic composition is not breast milk).

The expression **"infant formula"** as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula". In some embodiments, the infant formula is a preterm formula.

A **"follow-up formula"** or **"follow-on formula"** is given from the 6th month onwards. It constitutes the principal liquid element in the progressively diversified diet of this category of person.

The expression **"baby food"** means a foodstuff intended for particular nutritional use by infants or young children during the first years of life.

The expression **"infant cereal composition"** means a foodstuff intended for particular nutritional use by infants or young children during the first years of life.

The term **"fortifier"** refers to liquid or solid nutritional compositions suitable for mixing with breast milk or infant formula.

The term **"HMO"** or **"HMOs"** refers to human milk oligosaccharide(s). These carbohydrates are highly resistant to enzymatic hydrolysis, indicating that they may display essential functions not directly related to their caloric value. It has especially been illustrated that they play a vital role in the early development of infants and young children, such as the maturation of the immune system. Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy terminal positions at the nonreducing ends. The HMOs can be acidic (e.g. charged sialic acid containing oligosaccharide) or neutral (e.g. fucosylated oligosaccharide).

A **"fucosylated oligosaccharide"** is an oligosaccharide having a fucose residue. It has a neutral nature. Some examples are 2-FL (2'-fucosyllactose), 3-FL (3-fucosyllactose), difucosyllactose, lacto-N-fucopentaose (e.g. lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof. Without wishing to be bound by theory it is believed that the fucosyl-epitope of the fucosylated oligosaccharides may act as decoy at the mucosal surface.

The expressions **"fucosylated oligosaccharides comprising a 2'-fucosyl-epitope"** and **"2-fucosylated oligosaccharides"** encompass fucosylated oligosaccharides with a certain homology of form since they contain a 2'-fucosyl-epitope, therefore a certain homology of function can be expected.

The expression **"N-acetylated oligosaccharide(s)"** encompasses both "N-acetyl-lactosamine" and "oligosaccharide(s) containing N-acetyl-lactosamine". They are neutral oligosaccharides having an N-acetyl-lactosamine residue. Suitable examples are LNT (lacto-N-tetraose), para-lacto-N-neohexaose (para-LNnH), LNnT (lacto-N-neotetraose) and any combinations thereof. Other examples are lacto-N-hexaose, lacto-N-neohexaose, para- lacto-N-hexaose, para-lacto-N-neohexaose, lacto-N-octaose, lacto-N- neooctaose, iso- lacto-N-octaose, para- lacto-N-octaose and lacto-N-decaose.

The expression "at least one fucosylated oligosaccharide" and "at least one N-acetylated oligosaccharide" means "at least one type of fucosylated oligosaccharide" and "at least one type of N-acetylated oligosaccharide".

A **"precursor of HMO"** is a key compound that intervenes in the manufacture of HMO, such as sialic acid and/or fucose.

A **"sialylated oligosaccharide"** is a charged sialic acid containing oligosaccharide, i.e. an oligosaccharide having a sialic acid residue. It has an acidic nature. Some examples are 3-SL (3' sialyllactose) and 6-SL (6' sialyllactose).

The nutritional composition of the present invention can be in solid form (e.g. powder) or in liquid form. The amount of the various ingredients (e.g. the oligosaccharides) can be expressed in **g/100g** of composition on a dry weight basis when it is in a solid form, e.g. a powder, or as a concentration in **g/L** of the composition when it refers to a liquid form (this latter also encompasses liquid composition that may be obtained from a powder after reconstitution in a liquid such as milk, water..., e.g. a reconstituted infant formula or a follow-on/follow-up formula or an infant cereal product or any other formulation designed for infant nutrition). They can also be expressed in **g/100 kcal.**

The expression **"weaning period"** means the period during which the mother's milk is substituted by other food in the diet of an infant or young child.

The expressions **"X days/weeks/months/years of age", "X days/weeks/months/years of life"** and **"X days/weeks/months/years of birth"** can be used interchangeably.

The **"mother's milk"** should be understood as the breast milk or colostrum of the mother. HBM refers to Human Breast Milk.

The expressions **"infants/young children fed exclusively with human breast milk", "infants or young children exclusively breast fed", "exclusive breast fed infants or young children"and "breast-fed infants/young children"** can be used interchangeably. They refer to infants or young children fed with a great majority (i.e. at least 90%, or at least 95%, or at least 99%) or all (100%) of nutrients and/or energy originating from human breast milk.

The expression **"infants or young children exclusively fed nutritional compositions"** refers to infants or young children fed with a great majority (i.e. at least 90%, or at least 95%, or at least 99%) or all (100%) of nutrients and/or energy originating from synthetic nutritional compositions such as infant formula, follow-up milks or growing-up milks. The expression **"infants or young children predominantly fed nutritional compositions"** refers to infants or young children fed with nutritional sources of nutrients and/or energy predominantly originating from synthetic nutritional compositions such as infant formula, follow-up milks or growing-up milks. Predominantly refers to at least 50% (or at least 60% or at least 75%) of those nutrients and/or energy, such as from 50% to 90%, or from 60% to 80%.

The expression "**promoting and/or inducing**" in infants or young children a particular global microbiota in the gut refers to the development, the increase, the establishment, the apparition and/or the shifting of a particular global microbiota in said infants or young children.

The expression "**conventional nutritional composition**" refers to standard synthetic nutritional compositions such as infant formula, follow-up milks or growing-up milks already found in the market. A "conventional nutritional composition not comprising said oligosaccharides" refers to a standard nutritional composition that does not comprise the "at least one fucosylated oligosaccharide and at least one N-acetylated oligosaccharide".

The terms "**microbial**", "**microflora**" and "**microbiota**" can be used interchangeably.

The expressions "**microbiota in the gut**", "**microbiota of the gut**", "**gut microbiota**" and "**intestinal microbiota**" can be used interchangeably.

The terms "global", "overall", "whole", "entire" and "total" can be used interchangeably, especially in the expression "global microbiota". The expression "**global microbiota in/of the gut**" refers to the overall (or entire, whole, total) microbiota in the gut. It encompasses:
- the global microbiota composition, i.e. the relative taxonomic abundance (or amount) and/or the diversity of the entire microbiota in the gut, that is to say the "quantitative" and/or the "qualitative" aspects of this microbiota; and/or
- the global microbiota function, i.e. the activity and/or functionality of the entire microbiota in the gut, especially the metabolic activity/functionality. It may be assessed by measuring the relative abundance of predicted genes by metagenomics, or by quantitative profiling of major metabolites, including amino acids, major organics acids (lactate, succinate, citrate...) and/or carbohydrates.

A suitable and healthy gut microbiota is a key factor in the development of the mucosal immune system of the infant.

The expression "**gut microbiota dysbiosis**" refers to microbial imbalance in the gut.

The expression **"preventing and/or treating gut microbiota dysbiosis"** encompasses one or several of the following:
- preventing microbiota dysbiosis in the gut
- treating microbiota dysbiosis in the gut
- preventing and treating microbiota dysbiosis in the gut.

The expressions "down regulation" and "reduction" can be used interchangeably.

By the expressions "preventing" or "prevention", it is meant avoiding that a physical state, a condition or their consequences occurs and/or decreasing its incidence (i.e. reduction of the frequency).

By the expressions "treating" or "treatment", it is meant a decrease of the duration and/or of the severity of a physical state, a condition or their consequences.

The prevention and/or the treatment of a physical state, a condition or their consequences can occur during the treatment (i.e. during the administration of the composition of the present invention, either immediately after the start of the administration or some time after, e.g. some days or weeks after the start). But it can also encompass the prevention and/or the treatment later in life. The term "later in life" encompasses the effect after the termination of the intervention or treatment. The effect "later in life" can be from 1 week to several months, for example from 2 to 4 weeks, from 2 to 6 weeks, from 2 to 8 weeks, from 1 to 6 months or from 2 to 12 months.

The term **"prebiotic"** means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J Nutr. 1995;125:1401-12)*.*

The term **"probiotic"** means microbial cell preparations or components of microbial cells with a beneficial effect on the health or well-being of the host. (Salminen S, Ouwehand A. Benno Y. et al. "Probiotics: how should they be defined" Trends Food Sci. Technol. 1999:10 107-10). The microbial cells are generally bacteria or yeasts.

The term "**cfu**" should be understood as colony-forming unit.

All percentages are by weight unless otherwise stated.

In addition, in the context of the invention, the terms "comprising" or "comprises" do not exclude other possible elements. The composition of the present invention, including the many embodiments described herein, can comprise, consist of, or consist essentially of the essential elements and limitations of the invention described herein, as well as any additional or optional ingredients, components, or limitations described herein or otherwise depending on the needs.

Any reference to prior art documents in this specification is not to be considered an admission that such prior art is widely known or forms part of the common general knowledge in the field.

The invention will now be described in further details. It is noted that the various aspects, features, examples and embodiments described in the present application may be compatible and/or combined together.

A first object of the present invention is therefore a nutritional composition comprising at least one fucosylated oligosaccharide and at least one N-acetylated oligosaccharide for use in promoting and/or inducing in infants or young children a global microbiota in the gut that is closer to the global microbiota in the gut of infants or young children fed exclusively with human breast milk, in comparison to the global microbiota in the gut of infants or young children fed predominantly or exclusively with a conventional nutritional composition not comprising said oligosaccharides.

The nutritional composition of the present invention comprises at least one fucosylated oligosaccharide. There can be one or several types of fucosylated oligosaccharide(s). The fucosylated oligosaccharide(s) can indeed be selected from the list comprising 2'-fucosyllactose, 3'fucosyllactose, difucosyllactose, lacto-N-fucopentaose (such as lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose (such as fucosyllacto-N-neohexaose I, fucosyllacto-N-neohexaose II), difucosyllacto-N-hexaose I, difuco-lacto-N-neohexaose, difucosyllacto-N-neohexaose I, difucosyllacto-N-neohexaose II, fucosyl-para-Lacto-N-hexaose, tri-fuco-para-Lacto-N-hexaose I and any combination thereof.

In some particular embodiments the fucosylated oligosaccharide comprises a 2'-fucosyl-epitope. It can be for example selected from the list comprising 2'-fucosyllactose, difucosyllactose, lacto-N-fucopentaose, lacto-N-fucohexaose, lacto-N-difucohexaose, fucosyllacto-N-hexaose, fucosyllacto-N-neohexaose, difucosyllacto-N-hexaose difuco-lacto-N-neohexaose, difucosyllacto-N-neohexaose, fucosyl-para-Lacto-N-hexaose and any combination thereof.

In a preferred embodiment, the nutritional composition according to the invention comprises 2'-fucosyllactose (or 2FL, or 2'FL, or 2-FL or 2'-FL). In a particular embodiment, there is no othertype of fucosylated oligosaccharide than 2'-fucosyllactose, i.e. the nutritional composition of the invention comprises only 2'-fucosyllactose as fucosylated oligosaccharide.

The fucosylated oligosaccharide(s) may be isolated by chromatography or filtration technology from a natural source such as animal milks. Alternatively, it may be produced by biotechnological means using specific fucosyltransferases and/or fucosidases either through the use of enzyme-based fermentation technology (recombinant or natural enzymes) or microbial fermentation technology. In the latter case, microbes may either express their natural enzymes and substrates or may be engineered to produce respective substrates and enzymes. Single microbial cultures and/or mixed cultures may be used. Fucosylated oligosaccharide formation can be initiated by acceptor substrates starting from any degree of polymerization (DP), from DP = 1 onwards. Alternatively, fucosylated oligosaccharides may be produced by chemical synthesis from lactose and free fucose. Fucosylated oligosaccharides are also available for example from Kyowa, Hakko, Kogyo of Japan.

The nutritional composition of the present invention also comprises at least one the N-acetylated oligosaccharide. There can be one or several types of N-acetylated oligosaccharide. The N-acetylated oligosaccharide(s) can be for example lacto-N-tetraose (LNT), lacto-N-neotetraose (LNnT) or any combination thereof. In some particular embodiments the N-acetylated oligosaccharide is lacto-N-neotetraose (LNnT), para-lacto-N-neohexaose (para-LNnH) or any combination thereof. In some particular embodiments the N-acetylated oligosaccharide is LNnT. In some particular embodiments the N-acetylated oligosaccharide is LNT. In some other particular embodiments the N-acetylated oligosaccharide is a mixture of LNT and LNnT. In some particular embodiments the composition comprises both LNT and LNnT in a ratio LNT:LNnT between 5:1 and 1:2, or from 2:1 to 1:1, or from 2:1.2 to 2:1.6.

In a preferred embodiment, the nutritional composition according to the invention comprises lacto-N-neotetraose (LNnT). In a particular embodiment, there is no othertype of N-acetylated oligosaccharide than lacto-N-neotetraose (LNnT), i.e. the nutritional composition of the invention comprises only lacto-N-neotetraose (LNnT) as N-acetylated oligosaccharide.

The N-acetylated oligosaccharide(s) may be synthesised chemically by enzymatic transfer of saccharide units from donor moieties to acceptor moieties using glycosyltransferases as described for example in US patent No. 5,288,637 and WO 96/10086. Alternatively, LNT and LNnT may be prepared by chemical conversion of Keto-hexoses (e.g. fructose) either free or bound to an oligosaccharide (e.g. lactulose) into N-acetylhexosamine or an N-acetylhexosamine-containing oligosaccharide as described in Wrodnigg, T.M.; Stutz, A.E. (1999) Angew. Chem. Int. Ed. 38:827-828. N-acetyl-lactosamine produced in this way may then be transferred to lactose as the acceptor moiety.

In a particularly advantageous embodiment of the present invention, the nutritional composition comprises 2'-fucosyllactose (2FL) and lacto-N-neotetraose (LNnT).

In another specific embodiment, the nutritional composition of the present invention comprises an oligosaccharide mixture that consists of 2'-fucosyllactose (2-FL) and lacto-N-neotetraose (LNnT). In other words, the nutritional composition of the invention comprises only 2'-fucosyllactose (2-FL) as fucosylated oligosaccharide and only lacto-N-neotetraose (LNnT) as N-acetylated oligosaccharide.

The fucosylated oligosaccharide(s) can be present in the nutritional composition according to the present invention in a total amount of 0.8-1.5 g/L of the composition. In some embodiments, the fucosylated oligosaccharide(s) may be in a total amount of 0.85-1.3 g/L of the composition, such as 0.9-1.25 g/L or 0.9-1.1 g/L or 1-1.25 g/L or 1-1.2 g/L of the composition.

The fucosylated oligosaccharide(s) can be present in the nutritional composition in a total amount of 0.55-1.05 g/100g of composition on a dry weight basis. The fucosylated oligosaccharide(s) may be in a total amount of 0.66-1 g/100g of the composition, such as 0.70-0.97 g/100g or 0.70-0.85 g/100g or 0.78-0.97 g/100g or 0.78-0.93 g/100g of the composition.

The N-acetylated oligosaccharide(s) can be present in the nutritional composition according to the present invention in a total amount of 0.5-0.8 g/L of the composition.

In some embodiments, the N-acetylated oligosaccharide(s) may be in a total amount of 0.5-0.75 g/L or 0.5-0.7 g/L or 0.5-0.6 g/L of the composition.

The N-acetylated oligosaccharide(s) can be present in the nutritional composition in a total amount of 0.39-0.62 g/100g of composition on a dry weight basis, such as 0.39-0.58 g/100g or 0.39-0.54 g/100g or 0.39-0.47 g/100g.

These different ranges can all be combined together.

Therefore in one embodiment of the present invention, the nutritional composition comprises at least one fucosylated oligosaccharide and at least one N-acetylated oligosaccharide wherein:
- the fucosylated oligosaccharide(s) is/are in a total amount of 0.8-1.5 g/L of the composition and/or in a total amount of 0.62-1.16 g/100g of composition on a dry weight basis; and/or
- the N-acetylated oligosaccharide(s) is/are in a total amount of 0.5-0.8 g/L of the composition and/or in a total amount of 0.39-0.62 g/100g of composition on a dry weight basis.

In another particular embodiment the nutritional composition of the present invention comprises at least one fucosylated oligosaccharide and at least one N-acetylated oligosaccharide wherein:
- the fucosylated oligosaccharide(s) is/are in a total amount of 0.9-1.25 g/L of the composition and/or in a total amount of 0.70-0.97 g/100g of composition on a dry weight basis; and/or
- the N-acetylated oligosaccharide(s) is/are in a total amount of 0.5-0.7 g/L of the composition and/or in a total amount of 0.39-0.54 g/100g of composition on a dry weight basis.

In another particular embodiment the nutritional composition of the present invention comprises at least one fucosylated oligosaccharide and at least one N-acetylated oligosaccharide wherein:
- the fucosylated oligosaccharide(s) is/are in a total amount of 1-1.2 g/L of the composition and/or in a total amount of 0.78-0.93 g/100g of composition on a dry weight basis; and/or
- the N-acetylated oligosaccharide(s) is/are in a total amount of 0.5-0.6 g/L of the composition and/or in a total amount of 0.39-0.47 g/100g of composition on a dry weight basis.

The fucosylated oligosaccharide(s) and the N-acetylated oligosaccharide(s) comprised in the nutritional composition according to the invention are typically present in a ratio fucosylated oligosaccharide(s): N-acetylated oligosaccharide(s) of from 2:0.54 to 2:2.26, such as 2:0.76 - 2:1.8 or 2:0.8 - 2:1.4. In a particularly advantageous embodiment, this ratio is 2:1 or around 2:1.

The nutritional composition according to the present invention may also comprise at least another oligosaccharide(s) (i.e. other than the fucosylated oligosaccharide(s) and N-acetylated oligosaccharide(s) necessarily present in the composition) and/or at least a fiber(s) and/or at least a precursor(s) thereof. The other oligosaccharide and/or fiber and/or precursor thereof may be selected from the list comprising galacto-oligosaccharides (GOS), fructo-oligosaccharides (FOS), inulin, xylooligosaccharides (XOS), polydextrose, sialylated oligosaccharides, sialic acid, fucose and any combination thereof. They may be in an amount between 0 and 10% by weight of composition.

Suitable commercial products that can be used in addition to the oligosaccharides comprised in the oligosaccharide mixture to prepare the nutritional compositions according to the invention include combinations of FOS with inulin such as the product sold by BENEO under the trademark Orafti, or polydextrose sold by Tate & Lyle under the trademark STA-LITE®.

In a particular embodiment, the nutritional composition according to the invention can comprise at least about 0.4 g or at least 0.7 g of oligofructose per 100 kcal of the composition such as from about 0.4 to about 0.9 g, from about 0.4 to about 0.7 g, from about 0.4 to about 0.5 g, from about 0.7 to about 0.8 g, or from about 0.7 to about 0.9 g oligofructose per 100 kcal.

In some embodiments the oligofructose has a degree of polymerization of from 2 to 10. In some embodiments, at least 80%, 90%, 95%, 99% or 100% of the oligofructose has a degree of polymerization of from 2 to 8 (between 2 and 8).

In a particular embodiment, the nutritional composition according to the invention can comprise GOS. A galacto-oligosaccharide is an oligosaccharide comprising two or more galactose molecules which has no charge and no N-acetyl residue. Suitable galacto-oligosaccharides that may also be added in the nutritional composition according to the present invention include Galβ1,3Galβ1,4Glc, Galβ1,6Galβ1,4Glc, Galβ1,3Galβ1,3Galβ1,4Glc, Galβ1,6Galβ1,6Galβ1,4Glc, Galβ1,3Galβ1,6Galβ1,4Glc, Galβ1,6Galβ1,3Galβ1,4Glc, Galβ1,6Galβ1,6Galβ1,6Glc, Galβ1,3Galβ1,3Glc, Galβ1,4Galβ1,4Glc and Galβ1,4Galβ1,4Galβ1,4Glc but also any mixture thereof. Synthesized galacto-oligosaccharides such as Galβ1,6GaIβ1,4Glc, Galβ1,6Galβ1,6Galβ1,6Glc, Galβ1,3Galβ1,4Glc, Galβ1,6Galβ1,6Galβ1,4Glc, Galβ1,6GaIβ1,3Galβ1,4Glc, Galβ1,3Galβ1,6Galβ1,4Glc, Galβ1,4Galβ1,4Glc and Galβ1,4Galβ1,4Galβ1,4Glc and mixture thereof are commercially available under trademarks Vivinal® and Elix'or®. Other suppliers of oligosaccharides are Dextra Laboratories, Sigma-Aldrich Chemie GmbH and Kyowa Hakko Kogyo Co., Ltd. Alternatively, specific glycotransferases, such as galoctosyltransferases may be used to produce neutral oligosaccharides.

In a particular embodiment, the nutritional composition can also contain at least one bovine milk oligosaccharide. Conventional technologies for fractioning and enriching bovine milk fractions in bovine milk derived oligosaccharides can be used (such conventional technologies include column filtration, resin-filtration, nano-filtration, enzymatic treatment specially with beta-galactosidase, precipitation of proteins, crystallisation and separation of lactose etc,...). Some fractions of bovine milk enriched in oligosaccharides are commercially available or have been described for example in EP2526784 A1.

In a particular embodiment, the nutritional composition may also additionally comprise an oligosaccharide mixture ("BMOS") that comprises from 0.1 to 4.0 wt% of N-acetylated oligosaccharide(s), from 92.0 to 98.5 wt% of the galacto-oligosaccharide(s) and from 0.3 to 4.0 wt% of the sialylated oligosaccharide(s).

In a particular embodiment, the nutritional composition according to the invention can comprise sialylated oligosaccharide(s). There can be one or several sialylated oligosaccharide(s).

The sialylated oligosaccharide(s) can be selected from the group comprising 3' sialyllactose (3-SL), 6' sialyllactose (6-SL), and any combination thereof. In some embodiments of the invention the composition comprises 3-SL and 6-SL. In some particular embodiments the ratio between 3'-sialyllactose (3-SL) and 6'-sialyllactose (6-SL) can be in the range between 5:1 and 1:10, or from 3:1 and 1:1, or from 1:1 to 1:10. In some specific embodiments the sialylated oligosaccharide of the composition is 6' sialyllactose (6-SL).

The sialylated oligosaccharide(s) may be isolated by chromatographic or filtration technology from a natural source such as animal milks. Alternatively, they may be produced by biotechnological means using specific sialyltransferases or sialidases, neuraminidases, either by an enzyme based fermentation technology (recombinant or natural enzymes), by chemical synthesis or by a microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP), from DP=1 onwards. Alternatively, sialyllactoses may be produced by chemical synthesis from lactose and free N'-acetylneuraminic acid (sialic acid). Sialyllactoses are also commercially available for example from Kyowa Hakko Kogyo of Japan.

In particular examples the composition may comprise from 0.05 to 5 g/L of sialylated oligosaccharide(s), or from 0.1 to 4 g/L, or from 0.3 to 2 g/L, or from 0.4 to 1.5 g/L, or from 0.4 to 1 g/L, for example 0.5 or 0.9 g/L of sialylated oligosaccharide(s). In some particular embodiments the composition can comprise from 0.8 to 1.7 g/l of sialylated oligosaccharide(s).

The composition according to the invention can contain from 0.03 to 3.88 g of sialylated oligosaccharide(s) per 100g of composition on a dry weight basis, e.g. 0.08-3.10 g or 0.23-1.55 g or 0.31-1.16 g or 0.31-0.77 g or 0.39-0.7 g or 0.62-1.32 g of sialylated oligosaccharide(s) per 100g of composition on a dry weight basis.

In some particular embodiments of the present invention, the nutritional composition comprises sialylated oligosaccharide(s) in an amount of below 0.1g/100 g of composition on a dry weight basis.

In some particular embodiments of the present invention, the nutritional composition does not contain any sialylated oligosaccharide(s).

The composition according to the present invention may optionally also comprise at least one precursor of oligosaccharide. There can be one or several precursor(s) of oligosaccharide. For example the precursor of human milk oligosaccharide is sialic acid, fucose or a mixture thereof. In some particular embodiments the composition comprises sialic acid.

In particular examples the composition comprises from 0 to 3 g/L of precursor(s) of oligosaccharide, or from 0 to 2 g/L, or from 0 to 1 g/L, or from 0 to 0.7 g/L, or from 0 to 0.5 g/L or from 0 to 0.3 g/L, or from 0 to 0.2 g/L of precursor(s) of oligosaccharide.

The composition according to the invention can contain from 0 to 2.1 g of precursor(s) of oligosaccharide per 100g of composition on a dry weight basis, e.g. from 0 to 1.5 g or from 0 to 0.8 g or from 0 to 0.15 g of precursor(s) of oligosaccharide per 100g of composition on a dry weight basis.

The nutritional composition of the present invention can further comprise at least one probiotic (or probiotic strain), such as a probiotic bacterial strain.

The probiotic microorganisms most commonly used are principally bacteria and yeasts of the following genera: *Lactobacillus spp., Streptococcus spp., Enterococcus spp., Bifidobacterium spp.* and *Saccharomyces spp.*

In some particular embodiments, the probiotic is a probiotic bacterial strain. In some specific embodiments, it is particularly *Bifidobacteria* and/or *Lactobacilli.*

Suitable probiotic bacterial strains include *Lactobacillus rhamnosus* ATCC 53103 available from Valio Oy of Finland under the trademark LGG, *Lactobacillus rhamnosus* CGMCC 1.3724, *Lactobacillus paracasei* CNCM I-2116, *Lactobacillus johnsonii* CNCM I-1225, *Streptococcus salivarius* DSM 13084 sold by BLIS Technologies Limited of New Zealand under the designation KI2, *Bifidobacterium lactis* CNCM 1-3446 sold *inter alia* by the Christian Hansen company of Denmark under the trademark Bb 12, *Bifidobacterium longum* ATCC BAA-999 sold by Morinaga Milk Industry Co. Ltd. of Japan under the trademark BB536, *Bifidobacterium breve* sold by Danisco under the trademark Bb-03, *Bifidobacterium breve* sold by Morinaga under the trade mark M-16V, *Bifidobacterium infantis* sold by Procter & Gamble Co. under the trademark Bifantis and *Bifidobacterium breve* sold by Institut Rosell (Lallemand) under the trademark R0070.

In a particular embodiment the probiotic is a *Bifidobacterium lactis,* such as *Bifidobacterium lactis* CNCM 1-3446.

The nutritional composition according to the invention may contain from 10e3 to 10e12 cfu of probiotic strain, more preferably between 10e7 and 10e12 cfu such as between 10e8 and 10e10 cfu of probiotic strain per g of composition on a dry weight basis.

In one embodiment the probiotics are viable. In another embodiment the probiotics are non-replicating or inactivated. There may be both viable probiotics and inactivated probiotics in some other embodiments.

The nutritional composition of the invention can further comprise at least one phage (bacteriophage) or a mixture of phages, preferably directed against pathogenic Streptococci, Haemophilus, Moraxella and Staphylococci.

The nutritional composition according to the invention can be for example an infant formula, a starter infant formula, a follow-on or follow-up formula, a preterm formula, a baby food, an infant cereal composition, a fortifier such as a human milk fortifier, or a supplement. In some particular embodiments, the composition of the invention is an infant formula, a fortifier or a supplement that may be intended for the first 4 or 6 months of age. In a preferred embodiment the nutritional composition of the invention is an infant formula.

In some other embodiments the nutritional composition of the present invention is a fortifier. The fortifier can be a breast milk fortifier (e.g. a human milk fortifier) or a formula fortifier such as an infant formula fortifier or a follow-on/follow-up formula fortifier. When the nutritional composition is a supplement, it can be provided in the form of unit doses.

The nutritional composition of the present invention can be in solid (e.g. powder), liquid or gelatinous form.

The nutritional composition according to the invention generally contains a protein source. The protein can be in an amount of from 1.6 to 3 g per 100 kcal. In some embodiments, especially when the composition is intended for premature infants, the protein amount can be between 2.4 and 4 g/100kcal or more than 3.6 g/100kcal. In some other embodiments the protein amount can be below 2.0 g per 100 kcal, e.g. between 1.8 to 2.1 g/100kcal, or 1.8 - 2 g/100kcal or 1.9 - 2.1 g protein per 100 kcal, or in an amount below 1.8 g per 100 kcal such as 1.4-1.8 g/100kcal or 1.5-1.7 g/100kcal.

The type of protein is not believed to be critical to the present invention provided that the minimum requirements for essential amino acid content are met and satisfactory growth is ensured. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in any desired proportions. "Alpha-Lactalbumin" refers to a high-quality, easy-to-digest whey protein that comprises 20-25% of total human breast milk (HBM) protein and is the primary protein found in HBM. The structure of alpha-lactalbumin is comprised of 123 amino acids and 4 disulfide bridges and the protein has a molecular weight of 14.2K Daltons. Alpha-lactalbumin is ideal for lower protein infant formulas due to its high content of essential amino acids, particularly tryptophan. In one embodiment, the nutritional composition of this invention comprises alpha-lactalbumin in an amount of from about 0.2 to about 0.4 g/100 kcal of the nutritional composition, or in an amount of at least 1.7 g/L, or at least 2.0 g/L or at least 2.3 g/L, or at least 2.6 g/L of the nutritional composition.

In some advantageous embodiments the protein source is whey predominant (i.e. more than 50% of proteins are coming from whey proteins, such as 60% or 70%).

The proteins may be intact or hydrolysed or a mixture of intact and hydrolysed proteins. By the term "intact" is meant that the main part of the proteins are intact, i.e. the molecular structure is not altered, for example at least 80% of the proteins are not altered, such as at least 85% of the proteins are not altered, preferably at least 90% of the proteins are not altered, even more preferably at least 95% of the proteins are not altered, such as at least 98% of the proteins are not altered. In a particular embodiment, 100% of the proteins are not altered.

The term "hydrolysed" means in the context of the present invention a protein which has been hydrolysed or broken down into its component amino acids.

The proteins may be either fully (i.e. extensively) or partially hydrolysed. It may be desirable to supply partially hydrolysed proteins (degree of hydrolysis between 2 and 20%), for example for infants or young children believed to be at risk of developing cow's milk allergy. If hydrolysed proteins are required, the hydrolysis process may be carried out as desired and as is known in the art. For example, whey protein hydrolysates may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

In an embodiment of the invention at least 70% of the proteins are hydrolysed, preferably at least 80% of the proteins are hydrolysed, such as at least 85% of the proteins are hydrolysed, even more preferably at least 90% of the proteins are hydrolysed, such as at least 95% of the proteins are hydrolysed, particularly at least 98% of the proteins are hydrolysed. In a particular embodiment, 100% of the proteins are hydrolysed.

In one particular embodiment the proteins of the nutritional composition are hydrolyzed, fully hydrolyzed or partially hydrolyzed. The degree of hydrolysis (DH) of the protein can be between 8 and 40, or between 20 and 60 or between 20 and 80 or more than 10, 20, 40, 60, 80 or 90.

In a particular embodiment the nutritional composition according to the invention is a hypoallergenic composition. In another particular embodiment the composition according to the invention is a hypoallergenic nutritional composition.

The nutritional composition according to the present invention generally contains a carbohydrate source. This is particularly preferable in the case where the nutritional composition of the invention is an infant formula. In this case, any carbohydrate source conventionally found in infant formulae such as lactose, sucrose, saccharose, maltodextrin, starch and mixtures thereof may be used although one of the preferred sources of carbohydrates is lactose.

The nutritional composition according to the present invention generally contains a source of lipids. This is particularly relevant if the nutritional composition of the invention is an infant formula. In this case, the lipid source may be any lipid or fat which is suitable for use in infant formulae. Some suitable fat sources include palm oil, high oleic sunflower oil and high oleic safflower oil. The essential fatty acids linoleic and α-linolenic acid may also be added, as well small amounts of oils containing high quantities of preformed arachidonic acid and docosahexaenoic acid such as fish oils or microbial oils. The fat source may have a ratio of n-6 to n-3 fatty acids of about 5:1 to about 15:1; for example about 8:1 to about 10:1.

In one embodiment, the nutritional composition of this invention comprises triglycerides with high sn-2 palmitate, preferably triglycerides having more than 33% of the palmitic acids in sn-2 position.

In one embodiment, the nutritional composition of this invention comprises about 5 or 6 g per 100 kcal of fat, and for example at least about 7.5 wt% of this fat, for example, about 7.5-12.0%, consists of palmitic acid in the sn-2 position.

In one embodiment, of the invention the composition comprises at least 7.5%, preferably 8%, more preferably at least 9.6% of the fat is sn-2 palmitate, for example about 7.8-11.8%, about 8.0-11.5 wt%, about 8.5-11.0% or about 9.0-10.0 wt% of the fat is palmitic acid in the sn-2 position of a triglyceride.

In some embodiments, palmitic acid comprises from about 15 to about 25%, such as from about 15 to about 20%, of the total fatty acids content of the formula, by weight, and at least from about 30%, for example, from about 35 to about 43% of the total palmitic acid content is in the sn-2 position.

A commercially available composition sold by Lipid Nutrition is BetapolTM B-55, which is a triglyceride mixture derived from vegetable oil in which at least 54% of the palmitic acid is in the sn-2 position of the glycerol molecule. In one embodiment, the nutritional composition of the invention comprises a fat content that is about 40-50% BetapolTM B-55 by weight, for example, from about 43% to about 45% by weight. Those skilled in the art will appreciate that the percentage of the high sn-2 fat used and the total amount of sn-2 palmitate in the formula may vary, and that a different high sn-2 palmitate oil may be used, without departing from the spirit and scope of the invention.

The nutritional composition of the invention may also contain all vitamins and minerals understood to be essential in the daily diet and in nutritionally significant amounts. Minimum requirements have been established for certain vitamins and minerals. Examples of minerals, vitamins and other nutrients optionally present in the composition of the invention include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary depending on the intended population.

If necessary, the nutritional composition of the invention may contain emulsifiers and stabilisers such as soy, lecithin, citric acid esters of mono- and diglycerides, and the like.

The nutritional composition of the invention may also contain other substances which may have a beneficial effect such as lactoferrin, nucleotides, nucleosides, and the like.

The nutritional composition of the invention may also contain carotenoid(s). In some particular embodiments of the invention, the nutritional composition of the invention does not comprise any carotenoid.

The nutritional composition according to the invention may be prepared in any suitable manner. A composition will now be described by way of example.

For example, a formula such as an infant formula may be prepared by blending together the protein source, the carbohydrate source and the fat source in appropriate proportions. If used, the emulsifiers may be included at this point. The vitamins and minerals may be added at this point but they are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently in the range between about 50°C and about 80°C to aid dispersal of the ingredients. Commercially available liquefiers may be used to form the liquid mixture. The fucosylated oligosaccharide(s) and the N-acetylated oligosaccharide(s) may be added at this stage, especially if the final product is to have a liquid form. If the final product is to be a powder, they may likewise be added at this stage if desired.

The liquid mixture is then homogenised, for example in two stages.

The liquid mixture may then be thermally treated to reduce bacterial loads, by rapidly heating the liquid mixture to a temperature in the range between about 80°C and about 150°C for a duration between about 5 seconds and about 5 minutes, for example. This may be carried out by means of steam injection, an autoclave or a heat exchanger, for example a plate heat exchanger.

Then, the liquid mixture may be cooled to between about 60°C and about 85°C for example by flash cooling. The liquid mixture may then be again homogenised, for example in two stages between about 10 MPa and about 30 MPa in the first stage and between about 2 MPa and about 10 MPa in the second stage. The homogenised mixture may then be further cooled to add any heat sensitive components, such as vitamins and minerals. The pH and solids content of the homogenised mixture are conveniently adjusted at this point.

If the final product is to be a powder, the homogenised mixture is transferred to a suitable drying apparatus such as a spray dryer or freeze dryer and converted to powder. The powder should have a moisture content of less than about 5% by weight. The fucosylated oligosaccharide(s) and the N-acetylated oligosaccharide(s) may also or alternatively be added at this stage by dry-mixing or by blending them in a syrup form of crystals, along with the probiotic strain(s) (if used), and the mixture is spray-dried or freeze-dried.

If a liquid composition is preferred, the homogenised mixture may be sterilised then aseptically filled into suitable containers or may be first filled into the containers and then retorted.

In another embodiment, the composition of the invention may be a supplement.

The supplement may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, the supplement may contain an organic or inorganic carrier material suitable for oral or parenteral administration as well as vitamins, minerals trace elements and other micronutrients in accordance with the recommendations of Government bodies such as the USRDA.

The nutritional composition according to the invention is for use in infants or young children. It is particularly adapted for infants under 6 months of age.

The infants or young children may be born term or preterm. In a particular embodiment the nutritional composition of the invention is for use in infants or young children that were born preterm. In a particular embodiment the nutritional composition of the invention is for use in preterm infants.

In one embodiment, the nutritional composition of the present invention may also be used in an infant or a young child that was born small for gestational age or low birth weight.

Infants or young children with low birth weight may or may not be preterm, and similarly, infants or young children who are small for gestational age may or may not be preterm.

The nutritional composition of the present invention may also be used in an infant or a young child that was born by C-section or that was vaginally delivered.

All infants and young children can benefit from the invention as all of them are or can be, at a certain age, susceptible to acquiring an unbalanced intestinal/gut microbiota.

In some advantageous embodiments of the invention, the nutritional composition in for use infants or young children having a fragile or unbalanced microbiota or dysbiosis of microbiota, such as preterm infants, infants born by Caesarean-section, infants born small for gestational age or with low birth weight, hospitalized infants/young children, infants/young children treated or having been treated by antibiotics and/or infants/young children suffering or having suffered from gut infection and/or gut inflammation.

It is indeed foreseen that the composition of the invention may be even more beneficial to infants born with possibly impaired gut microbiota or fragile infants/young children (such as prematurely born infants and/or infants born by C-section). It is also foreseen that the composition of the invention may be even more beneficial to infants/young children exhibiting intestinal disorders (such as diarrhea, infections or colic), especially after birth, for example, during the first 4 weeks after birth.

In embodiments of the invention, the infants born prematurely or born by caesarean section or born small for gestational age or with low birth weight, or exhibiting unbalanced or abnormal gut microbiota or suffering or having suffered from gut infection and/or gut inflammation, are targeted by the composition of the present invention, and especially when the infants are 0-6 months of age. Without being bound by the theory, it is believed that younger infants benefit even more from the composition of the invention, especially when the infants have (or are at risk of having) an unbalanced intestinal microbiota and/or have a fragile health condition (as exemplified by the conditions cited above).

In such infants, acquiring a gut microbiota that is close to the gut microbiota of breast fed infant (preferably exclusively breast fed infants) is of particular interest. Indeed it provides them with a good number of health elements that can be beneficial, especially for those fragile infants.

The nutritional composition can be administered (or given or fed) at an age and for a period that depends on the needs.

In one embodiment, the infants or young children are 0-36 months of age, such as 0-12 months or 0-6 months of age. It is foreseen that the composition of the invention may be even more beneficial to infants just after birth (0-4 weeks or 0-8 weeks) as their intestinal tract may be more fragile.

In some particular embodiments, the nutritional composition can be an infant formula and may be especially intended for infants between 0 and 12 months of age fed predominantly with infant formula.

In some advantageous embodiments the nutritional composition can be for example given immediately after birth of the infants. The composition of the invention can also be given during the first week of life of the infant, or during the first 2 weeks of life, or during the first 3 weeks of life, or during the first month of life, or during the first 2 months of life, or during the first 3 months of life, or during the first 4 months of life, or during the first 6 months of life, or during the first 8 months of life, or during the first 10 months of life, or during the first year of life, or during the first two years of life or even more. In some particularly advantageous embodiments of the invention, the nutritional composition is given (or administered) to an infant within the first 4 or 6 months of birth of said infant. In some other embodiments, the nutritional composition of the invention is given few days (e.g. 1, 2, 3, 5, 10, 15, 20...), or few weeks (e.g. 1, 2, 3, 4, 5, 6, 7, 8, 9, 10...), or few months (e.g. 1, 2, 3, 4, 5, 6, 7, 8, 9, 10...) after birth.

The nutritional composition of the present invention may be given for some days (1, 2, 3, 4, 5, 6...), or for some weeks (1, 2, 3, 4, 5, 6, 7, 8 or even more), or for some months (1, 2, 3, 4, 5, 6, 7, 8, 9, 10, 11 or even more), depending on the needs.

In some embodiments the composition according to the invention can be for use before and/or during the weaning period.

In one embodiment the composition of the invention is given to the infant or young child as a supplementary composition to the mother's milk. In some embodiments the infant or young child receives the mother's milk during at least the first 2 weeks, or the first 1, 2, 4, or 6 months. In one embodiment the nutritional composition of the invention is given to the infant or young child after such period of mother's nutrition, or is given together with such period of mother's milk nutrition. In another embodiment the composition is given to the infant or young child as the sole or primary nutritional composition during at least one period of time, e.g. after the 1 st, 2nd or 4th month of life, during at least 1, 2, 4 or 6 months.

In one embodiment the nutritional composition of the invention is a complete nutritional composition (fulfilling all or most of the nutritional needs of the subject). In another embodiment the nutrition composition is a supplement or a fortifier intended for example to supplement human milk or to supplement an infant formula or a follow-on/follow-up formula.

The nutritional composition of the present invention has a positive effect on the overall microbiota of the subject infants or young children: it promotes and/or induces a global microbiota in the gut of the infants or young children fed with the nutritional composition of the present invention that is closer to the one of infants or young children fed exclusively with human breast milk, in comparison to the global microbiota in the gut of infants or young children fed predominantly or exclusively with a conventional nutritional composition not comprising said oligosaccharides (i.e. not comprising the at least one fucosylated oligosaccharide and the at least one N-acetylated oligosaccharide).

The major and surprising health benefit of the nutritional composition of the present invention is that it modulates the global microbiota of infants fed with the nutritional composition of the invention on a global way to bring it closer to that of breast-fed infants. Not only some specific taxa of the microbiota is changed but the nutritional composition especially induces a shift of the global microbiota composition towards the one induced by breast feeding. In addition, as illustrated by the experiments, both the gut microbiota composition (i.e. the relative taxonomic abundance and/or the diversity of the global microbiota) and the gut microbiota function (i.e. its activity and/or functionality, e.g. the resulting metabolites) gets closer to the breast-fed infants. So with regards to the gut microbiota composition, the induced/promoted microbiota is specific around 2 dimensions:
"quantitatively": the gut flora comprises more beneficial bacteria and less non-beneficial or detrimental bacteria;
"qualitatively": the variety of bacterial taxa resembles more to a microbiota of breast-fed infants.

The diversity of the global microbiota may be the alpha diversity (details are indicated in the example part), and it may for example be illustrated as measured by PD_whole_tree. The health effect provided by the nutritional composition of the invention may be measured in infants or young children between 0 and 36 months, optionally between 0 and 12 months of age. It can be observed after a few days or weeks of use of the composition - for example after 4 weeks or 6 weeks or 8 weeks of use. It may however take 4, 6, 8 weeks before the induced microbiota to be observed. The observation of this promoted/induced global microbiota can however take 4, 6, 8 weeks before been observable. It may for example be measured in the stools of the infants/young children. In the context of the invention, the health benefit brings the global microbiota of the gut of the infants or young children closer to the microbiota of exclusively breast-fed infants or young children. This is especially observed when comparing it to infants or young children not receiving the composition of the present invention.

Such a positive effect may comprise i) the down regulation, decrease or inhibition of growth of pathogenic bacteria or the reduction of the pathogenic bacteria load and/or ii) the up regulation, increase or promotion of growth of beneficial bacteria. In some embodiments, the nutritional composition of the present invention involves (or comprises or is accompanied by or is characterized by) an up-regulation of the population of *Bifidobacterium* and/or a down regulation of the populations of *Escherichia* and/or *Peptostreptococcaceae,* in comparison to the global microbiota in the gut of infants or young children fed predominantly or exclusively with a conventional nutritional composition. This effect can be measured in the stools of said infants or young children, for example at or after 1, 4, 6 or 8 weeks of age, and for example after 1, 4, 6 or 8 weeks of feeding with said nutritional composition. The stool microbiota composition may be measured for example based on 16S rDNA analysis or on metagenome analysis (details are indicated in the example part).

By down-regulating, decreasing and/or inhibiting the growth of populations of pathogenic bacteria, and/or inducing more beneficial bacteria, qualitatively and quantitatively, the composition of the invention provides positive health effects. Such a healthy gut/intestinal microbiota is ultimately linked to proper nutrient absorption, adequate growth, less colic, less infection, less diarrhea and a better gut health.

The effect of the invention can be preventive (for example avoiding the imbalance of the gut microbiota, avoiding gut infections, maintaining a healthy intestinal microbiota, inducing a healthy intestinal microbiota) or curative (restoring a healthy gut microbiota when it is impaired, helping eliminate or decrease pathogenic populations in the gut/ intestine, inducing a healthy microbiota after impairments due, for example, to diarrhea or infections).

In some embodiments, the nutritional composition of the present invention involves (or comprises or is accompanied by or is characterized by) a reduction of pathogen(s) and/or a reduction of virulence factor(s), in comparison to the global microbiota in the gut of infants or young children fed predominantly or exclusively with the conventional nutritional composition not comprising the oligosaccharides. These effects can be measured in the stools of said infants or young children, for example at or after 1, 4, 6 or 8 weeks of age, and for example after 1, 4, 6 or 8 weeks of feeding with said nutritional composition.

The reduction of pathogen(s) means a reduction of the pathogen(s) occurrence and/or amount (e.g. load, quantities). It can be measured for example by using the Luminex PCR method (details are indicated in the example part). The pathogens that may be reduced can be virus, bacteria and/or protists. Particular examples of viral pathogens are norovirus (e.g. Norovirus GI/GII) and/or rotavirus (e.g. Rotavirus A). Particular examples of bacterial pathogens are *Clostridium difficile* (e.g. *Clostridium difficile toxin A*/*B),* Campylobacter, *Escherichia coli* (e.g. E. *coli* 0157). Particular examples of protist pathogens are Cryptosporidium.

In a particular example, the nutritional composition involves a decrease of the bacterial pathogen *Clostridium difficile.*

The virulence factor(s) may be virulence genes and/or antibiotic resistance genes that can be detected for example by metagenome analysis (details are indicated in the example part). Particular examples of virulence genes are gi:16767513 (yjcB - putative inner membrane protein) from *Salmonella enterica* LT2, gi:21284341 (cna - collagen adhesin precursor) from *Staphylococcus aureus* MW2, gi:24528016 (I7045 - L7045) from *Escherichia coli* 536, gi:15808725 (fecB - FecB) from *Shigella flexneri* YSH6000 and gi:21282741 (isdA - cell surface protein) from *Staphylococcus aureus* MW2.

In some embodiments, the nutritional composition of the present invention involves (or comprises or is accompanied by or is characterized by) a reduction of the production of free amino acids and/or a stimulation of the production of lactate, in comparison to the global microbiota in the gut of infants or young children fed predominantly or exclusively with a conventional nutritional composition not comprising the oligosaccharides. Lactate is produced by lactic acid bacteria like lactobacillus and bifidobacteria, and it may prevent the growth of other bacteria including the pathogen ones. Particular examples of free amino acids are phenylalanine, tyrosine and isoleucine. These effects can be measured in the stools of said infants or young children, for example at or after 1, 4, 6 or 8 weeks of age, and for example after 1, 4, 6 or 8 weeks of feeding with said nutritional composition. The measures may be made using a well-established metabonomics approach based on proton Nuclear Magnetic Resonance Spectroscopy (1H NMR) (Moco et al, 2013, Metabolomics perspectives in pediatric research, Pediatr.Res. 73, 570-576).

The health effect provided to the infants or young children can be measured by various methods as illustrated in the example below.

In one embodiment the effect on the global microbiota is measured by calculating the alpha diversity of each sample and analyzing their distribution (details are indicated in the example part).

In one embodiment the effect on the global microbiota is measured by calculating the beta diversity between groups with a metric which takes into account the phylogenetic distances between the OTUs (Operational Taxonomic Units) for example the UniFrac method and analyzing their distribution. Alternatively, the beta-diversity between Control, Test, and Breast-fed groups may be evaluated by a multivariate ordination with hypothesis testing based on randomization procedures (e.g. Canonical Correspondence Analysis (CCA), Redundancy Analysis (RDA)), or a multivariate parametric or non-parametric test (e.g. Adonis, ANOSIM, multivariate ANOVA). In a particular example, the beta diversity is calculated using RDA.

In one embodiment of the invention, the promoted and/or inducted global microbiota in the gut of infants and/or young children feeding the nutritional composition of the invention has an alpha diversity significantly reduced (e.g. reduced of at least 0.10 units, such as at least 0.12 units or at least 0.15 units, for example 0.19 units) in comparison to the global microbiota in the gut of infants or young children fed predominantly or exclusively with a conventional nutritional composition not comprising the oligosaccharides present in the nutritional composition of the invention (e.g. the at least one fucosylated oligosaccharide and the at least one N-acetylated oligosaccharide), and therefore is closer to the one of the breast-fed infants..

A suitable and healthy gut microbiota is a key factor in the development of the mucosal immune system of the infant. The nutritional composition of the present invention may be used for prevention and/or treatment purposes.

In a particular aspect, the present invention also refers to a nutritional composition for use in providing a healthy growth, for use in providing a healthy immune system, for use in providing a healthy gut function and/or for use in preventing and/or treating gut microbiota dysbiosis in infants or young children.

The beneficial health benefits provided by the composition of the invention can be short term and/or long term effects.

The effect may be immediate with the administration of the composition of the present invention, or later in life, i.e. after the administration of the composition, e.g. from 1 week to several months, for example from 2 to 4 weeks, from 2 to 6 weeks, from 2 to 8 weeks, from 1 to 6 months or from 2 to 12 months after said administration.

### Other objects:

Another object of the present invention is the use of at least one fucosylated oligosaccharide and at least one N-acetylated oligosaccharide in the preparation of a nutritional composition for promoting and/or inducing in infants or young children a global microbiota in the gut that is closer to the global microbiota in the gut of infants or young children fed exclusively with human breast milk, in comparison to the global microbiota in the gut of infants or young children fed predominantly or exclusively with a conventional nutritional composition not comprising said oligosaccharides.

Another object of the present invention is the use of the nutritional composition according to the present invention for providing a healthy growth, for providing a healthy immune system, for providing a healthy gut function and/or for preventing and/or treating gut microbiota dysbiosis in infants or young children.

Another object of the present invention is a pharmaceutical composition comprising at least one fucosylated oligosaccharide and at least one N-acetylated oligosaccharide for promoting and/or inducing in infants or young children a global microbiota in the gut that is closer to the global microbiota in the gut of infants or young children fed exclusively with human breast milk, in comparison to the global microbiota in the gut of infants or young children fed predominantly or exclusively with a conventional nutritional composition not comprising said oligosaccharides.

This pharmaceutical composition may be used for providing a healthy growth, for providing a healthy immune system, for providing a healthy gut function and/or for preventing and/or treating gut microbiota dysbiosis in infants or young children.

Another object of the present invention refers to a method for promoting and/or inducing in infants or young children a global microbiota in the gut that is closer to the global microbiota in the gut of infants or young children fed exclusively with human breast milk, in comparison to the global microbiota in the gut of infants or young children fed predominantly or exclusively with a conventional nutritional composition not comprising said oligosaccharides, said method comprising administering to said infant or young child a nutritional composition comprising at least one fucosylated oligosaccharide and at least one N-acetylated oligosaccharide.

Another object of the present invention is a method for providing a healthy growth, for providing a healthy immune system, for providing a healthy gut function and/or for preventing and/or treating gut microbiota dysbiosis in infants or young children, said method comprising administering to said infant or young child a nutritional composition comprising at least one fucosylated oligosaccharide and at least one N-acetylated oligosaccharide.

The previously-mentioned embodiments and examples (e.g. related to the types and amounts of oligosaccharide, the nutritional composition, the administration, the targeted population...) also apply for these various objects (i.e. uses, pharmaceutical composition, methods...).

### Examples

The following examples illustrate some specific embodiments of the composition for use according to the present invention. The examples are given solely for the purpose of illustration and are not to be construed as limitations of the present invention, as many variations thereof are possible without departing from the spirit of the invention.

### Example 1

An example of the composition of a nutritional composition (e.g. an infant formula) according to the present invention is given in the below table 1. This composition is given by way of illustration only.

**Table 1: an example of the composition of a nutritional composition (e.g. an infant formula) according to the present invention**

| **Nutrients** | | **per 100kcal** | **per litre** |
|---|---|---|---|
| Energy (kcal) | | 100 | 670 |
| Protein (g) | | 1.83 | 12.3 |
| Fat (g) | | 5.3 | 35.7 |
| Linoleic acid (g) | | 0.79 | 5.3 |
| α-Linolenic acid (mg) | | 101 | 675 |
| Lactose (g) | | 11.2 | 74.7 |
| Minerals (g) | | 0.37 | 2.5 |
| Na (mg) | | 23 | 150 |
| K (mg) | | 89 | 590 |
| Cl(mg) | | 64 | 430 |
| Ca(mg) | | 62 | 410 |
| P (mg) | | 31 | 210 |
| Mg (mg) | | 7 | 50 |
| Mn (µg) | | 8 | 50 |
| Se (µg) | | 2 | 13 |
| Vitamin A (µg RE) | | 105 | 700 |
| Vitamin D (µg) | | 1.5 | 10 |
| Vitamin E (mg TE) | | 0.8 | 5.4 |
| Vitamin K1 (µg) | | 8 | 54 |
| Vitamin C (mg) | | 10 | 67 |
| Vitamin B1 (mg) | | 0.07 | 0.47 |
| Vitamin B2 (mg) | | 0.15 | 1.0 |
| Niacin (mg) | | 1 | 6.7 |
| Vitamin B6 (mg) | | 0.075 | 0.50 |
| Folic acid (µg) | | 9 | 60 |
| Pantothenic acid (mg) | | 0.45 | 3 |
| Vitamin B12 (µg) | | 0.3 | 2 |
| Biotin (µg) | | 2.2 | 15 |
| Choline (mg) | | 10 | 67 |
| Fe (mg) | | 1.2 | 8 |
| I (µg) | | 15 | 100 |
| Cu (mg) | | 0.06 | 0.4 |
| Zn (mg) | | 0.75 | 5 |
| Oligosaccharides (HMOs) | 2FL (g) | 0.15 | 1 |
| | LNnT (g) | 0.075 | 0.5 |

### Example 2

### Description of the clinical study

A safety trial was conducted at the Dipartimento Materno Infantile, Unità Operativa Complessa di Neonatologia e Terapia Intensiva Neonatale, AOUP "Paolo Giaccone" in Palermo, Italy and Kinderartsenpraktijk in Hasselt, Belgium.

This study was a randomized, controlled, two-center interventional clinical trial of 2 parallel formula-fed groups. The study population for the formula-fed groups consisted of healthy, full-term male and female infants old 0 to 14 days at enrolment who were exclusively formula-fed at the time of enrolment. Eligible infants were randomly assigned to one of two study formulas (Control or Test) using delivery method (vaginal or Caesarean section) and gender as stratification factors. For Stage 1, randomized infants received exclusive feedings with the Test or Control formulas from enrolment through 4 months of age in amounts suitable for their weight, age and appetite. Parents/caregivers, investigators, study support staff, and the Clinical Project Manager were blinded to the identity of the study formulas.

The infant formulas used in the study were as follows:
- A Control Formula was given to the Control group: it was a standard whey-predominant starter infant formula comprising LC-PUFA and without probiotics (66.9 kcal/100 ml reconstituted formula, 1.889 g protein/100 kcal powder with a whey:casein ratio of 71.6%:28.4%, see table 2 for the detailed composition).
- Test Formula was given to the Test group: it was the Control Formula except that a part of lactose has been replaced with 2 HMOs (2FL and LNnT) in the following amounts 0.5-0.6 g LNnT and 1.0-1.2 g 2'FL per liter of reconstituted formula (see table 2 for the detailed composition).

As reference group (Breast-fed group = BF group), at least for 3 months exclusively breast-fed infants were recruited for stool sampling at 3 months of age.

**Table 2: composition of the Control Formula and the Test Fomula**

| **Parameter** | | **100 g Control formula** | **100 g Test formula** |
|---|---|---|---|
| Energy (kcal) | | 518.7 | 518.7 |
| Water (g) | | 2.6 | 2.6 |
| Fat (g) | | 27.5 | 27.5 |
| Fatty acids saturated (g) | | 11 | 11 |
| Fatty acids Mono-unsaturated (g) | | 9.4 | 9.4 |
| Alpha-Linolenic Acid C18:3 n-3 (mg) | | 510 | 510 |
| Docosahexaenoic Acid C22:6 n-3 (DHA) (mg) | | 61 | 61 |
| Arachidonic Acid C20:4 n-6 (ARA) (mg) | | 61 | 61 |
| Linoleic Acid C18:2 n-6 (mg) | | 4270 | 4270 |
| Fatty acids Poly-unsaturated (g) | | 4.9 | 4.9 |
| Protein (g) | | 9.8 | 9.8 |
| Available Carbohydrates (g) | | 58 | 58 |
| Lactose (g) | | 56 | 55 |
| HMOs | LNnT(g) | 0 | 0.39-0.47 |
| | 2FL (g | 0 | 0.77-0.93 |
| Sugars (g) | | 56 | 56 |
| Ash (g) | | 2.1 | 2.1 |
| Sodium (mg) | | 162 | 162 |
| Potassium (mg) | | 575 | 575 |
| Chloride (mg) | | 348 | 348 |
| Calcium (mg) | | 317 | 317 |
| Phosphorus (mg) | | 178 | 178 |
| Magnesium (mg) | | 43 | 43 |
| Manganese (µg) | | 121 | 121 |
| Selenium (µg) | | 13 | 13 |
| Iron (mg) | | 4.8 | 4.8 |
| Copper (mg) | | 0.37 | 0.37 |
| Zinc (mg) | | 5 | 5 |
| Iodine (µg) | | 102 | 102 |
| Fluoride (µg) | | 60 | 60 |
| Vitamin A (Retinol) (µg RE) | | 542 | 542 |
| Vitamin D (Calciferol) (µg D) | | 7.6 | 7.6 |
| Vitamin E (Tocopherol) (mg TE) | | 8.4 | 8.4 |
| Vitamin K (Phytoquinone) (µg) | | 45 | 45 |
| Vitamin C (Ascorbic Acid) (mg) | | 80 | 80 |
| Vitamin B1 (Thiamin Base) (mg) | | 0.6 | 0.6 |
| Vitamin B2 (Riboflavin) (mg) | | 0.67 | 0.67 |
| Niacin (mg) | | 4.1 | 4.1 |
| Vitamin B6 (Pyridoxine Base) (mg) | | 0.34 | 0.34 |
| Folic acid (µg) | | 80.9 | 80.9 |
| Pantothenic Acid (mg) | | 3.4 | 3.4 |
| Vitamin B12 (Cyanocobalamin) (µg) | | 1.8 | 1.8 |
| Biotin (µg) | | 14 | 14 |
| Choline (mg) | | 48 | 48 |
| Inositol (mg) | | 48 | 48 |
| Taurine (mg) | | 33 | 33 |
| Carnitine, L-(mg) | | 9.5 | 9.5 |
| Nucleotides (mg) | | 15 | 15 |
| Adenosine 5'-Monophosphate (mg) | | 3.8 | 3.8 |
| Cytidine 5'-Monophosphate (mg) | | 6 | 6 |
| Guanosine 5'-Monophosphate (mg) | | 1.2 | 1.2 |
| Uridine 5'-Monophosphate (mg) | | 4 | 4 |
| Ca / P (ratio) | | 1.781 | 1.781 |
| Linoleic / Alpha-linolenic (ratio) | | 8.373 | 8.373 |
| Vitamin C / Fe (ratio) | | 16.667 | 16.667 |
| Phenylalanine, L-(mg) | | 488 | 488 |
| Alanine, L-(mg) | | 391 | 391 |
| Arginine, L-(mg) | | 258 | 258 |
| Cystine, L-(mg) | | 247 | 247 |
| Histidine, L-(mg) | | 226 | 226 |
| Isoleucine, L-(mg) | | 502 | 502 |
| Leucine, L- (mg) | | 1045 | 1045 |
| Methionine, L-(mg) | | 205 | 205 |
| Threonine, L-(mg) | | 460 | 460 |
| Tryptophan, L-(mg) | | 200 | 200 |
| Tyrosine, L-(mg) | | 399 | 399 |
| Valine, L-(mg) | | 542 | 542 |

Evaluation of stool microbiota were made for each group at 3 months of age and using different techniques, see table 3.

**Table 3: Number of infants in the intention to treat groups (ITT), per protocol groups (PP) and number of stool samples that were available from the per protocol groups or the breastfed reference group for microbiota analysis by global 16S rDNA sequencing, pathogen specific Luminex PCR amplification, global metagenome sequencing and NMR based metabolite profiling.**

| Nb of samples | ITT | PP | PP samples 16S rDNA | PP samples Luminex PCR | PP samples metagenome | PP samples metabolites |
|---|---|---|---|---|---|---|
| Control | 87 | 75 | 63 | 54 | 65 | 64 |
| Test | 88 | 71 | 58 | 50 | 58 | 57 |
| Breast-Fed | - | 38 | 33 | - | 34 | 32 |

### Materials and Methods

### Stool collection

### Stool samples were collected by parents from all subjects at home and within the 48 hours preceding the 3-month visit. To this end parents were supplied a kit (insulated bag, ice pack, spatula pots, sealable plastic bags, instruction sheet). Parents were asked to collect 2 samples, to store the samples at home in a -20°C freezer and to transport the stool samples within the insulated bag containing a frozen ice pack to the site of the visit where samples were kept frozen at -80°C. Samples were then shipped to the Nestle Research Center, Switzerland, on dry ice and kept frozen at -80°C until analysis.

### Fecal DNA extraction

Total DNA was extracted using the QIAamp DNA Stool Mini Kit (QIAGEN), following the manufacturer's instructions, except for the addition of a series of mechanical disruption steps (11 × 45 s) using a FastPrep apparatus and Lysing Matrix B tubes (MP Biochemicals) (Junick and Blaut, 2012, Quantification of human fecal bifidobacterium species by use of quantitative real-time PCR analysis targeting the groEL gene. Appl Environ Microbiol 78: 2613-2622).

### Amplification of 16S genes and sequencing

Then, the 16S variable region V3 to V4 were PCR amplified using universal (Klindworth et al., 2013, Evaluation of general 16S ribosomal RNA gene PCR primers for classical and next-generation sequencing-based diversity studies. Nucleic Acids Res 41: e1) and sequenced with Illumina Miseq technology as previously described (Caporaso et al., 2012, Ultra-high-throughput microbial community analysis on the Illumina HiSeq and MiSeq platforms. ISME J 6: 1621-1624).

### 16S data analysis

After quality filtering, 6'710'039 sequences described the microbiota of 154 samples of the per protocol (PP) set (see Table 3), with an average coverage of 42'739 sequences per sample classified in 173 OTUs. Three samples with less than 10'000 sequences were excluded from the 16S rDNA analysis.

Raw sequence data were analyzed using a blend of Mothur (Schloss et al., 2009, Introducing mothur: open-source, platform-independent, community-supported software for describing and comparing microbial communities. Appl Environ Microbiol 75: 7537-7541) and QIIME (Caporaso et al., 2010, QIIME allows analysis of high-throughput community sequencing data. Nat Methods 7: 335-336) software packages. Paired-end sequences were demultiplexed and joined as described (Kozich et al., 2013 Development of a dual-index sequencing strategy and curation pipeline for analyzing amplicon sequence data on the MiSeq Illumina sequencing platform. Appl Environ Microbiol 79: 5112-5120). Then, sequences were splitted in separated fasta files for each sample using Mothur commands [deunique.seqs(), degap.seqs(), and split.groups()]. Conversion to QIIME format using add_qiime_labels.py and subsequent analytical steps were performed in QIIME. Chimera check and OTUs picking at 97% identity were performed using Uchime (Edgar et al., 2011, UCHIME improves sensitivity and speed of chimera detection. Bioinformatics 27: 2194-2200) with pick_open_reference.py. Taxonomy assignment was performed on representative sequences using RDP Classifier with confidence threshold of 0.6 (Wang et al., 2007, Naive Bayesian classifier for rapid assignment of rRNA sequences into the new bacterial taxonomy. Appl Environ Microbiol 73: 5261-5267). OTU representative sequences were aligned using PyNast method (Caporaso et al., 2010, PyNAST: a flexible tool for aligning sequences to a template alignment. Bioinformatics 26: 266-267) and Uclust as pairwise alignment method. The resulting multiple alignments was then filtered and used to build a phylogenetic tree with the FastTree method (Price et al., 2009, FastTree: computing large minimum evolution trees with profiles instead of a distance matrix. Mol Biol Evol 26: 1641-1650). After quality filtering (Bokulich et al., 2013, Quality-filtering vastly improves diversity estimates from Illumina amplicon sequencing. Nat Methods 10: 57-59), alpha diversity analyses were performed in QIIME and Redundancy analysis (RDA) (Kindt R and Coe R., 2005, Tree diversity analysis. A manual and software for common statistical methods for ecological and biodiversity studies. Nairobi: World Agroforestry Centre - ICRAF) at genus level with the websites Calypso at http://bioinfo.qimr.edu.au/calypso.

### Metagenome analysis

The microbial composition of the stool samples of the three groups was determined by multiplexed high-throughput sequencing of DNA isolated from stool using Illumina HiSeq instrument with PE 100 reads. DNA libraries were produced with the Nextera XT protocol. The samples were sequenced on 6 high output Flow Cell.

First reads were trimmed using DynamicTrim (v2.1) from the SolexaQA package (Cox, M.P. et al., BMC Bioinformatics, 2010, SolexaQA: At-a-glance quality assessment of Illumina second-generation sequencing data. BMC Bioinformatics 11:485-490) at a probability cutoff of 0.05. The resulting trimmed sequences were then filtered at a length threshold of 25bp using LengthSort (v2.1) from the SolexaQA package. Filtered reads were mapped against the complete human genome hg19 using bowtie v2.2.5 (Langmead, B. and Salzberg, S., Nature Methods., 2012, Fast gapped-read alignment with Bowtie 2. Nature Methods 9:357-359) to remove human reads. On average 0.9, 0.02 and 3.3% human reads were identified in the Control, Test and Breast-fed group respectively. In order to increase computational efficiency the number of reads were further reduced using the unique.seqs function from mothur v1.35 (Schloss, P.D., et al., Appl Environ Microbiol, 2009, Introducing mothur: open-source, platform-independent, community-supported software for describing and comparing microbial communities. Appl Environ Microbiol 75: 7537-7541), which returns only the unique sequences found. This step reduced the number of reads by -50%. After quality filtering, a median number of 76 to 80 million sequences were obtained of 157 samples of the per protocol (PP) set (see table 3) with an equal coverage of the Control, Test and BF reference group. A median of 36 to 42 million unique sequences were obtained again equally distributed between groups. One sample with only 53661 sequences was excluded from the metagenome analysis.The remaining reads were then used to profile the composition of microbial communities using MetaPhlAn v2 (Segata, N. et al., Nature Methods, 2012, Metagenomic microbial community profiling using unique clade-specific marker genes. Nature Methods 9:811-814).

The presence of genes encoding known virulence factors or antibiotic resistance genes was studied using ShortBRED. ShortBRED is a pipeline that takes a set of protein sequences, groups them into families, extracts a set of distinctive strings ("markers"), and then searches for these markers in metagenomic data and determines the presence and abundance of the protein families of interest. The markers for virulence factors were based on all protein sequences (2447 protein sequence) from the R3 release of the Virulence Factor of Pathogenic Bacteria Database (http://www.mgc.ac.cn/VFs/ - Chen, L.H. et al., 2012, Toward the genetic diversity and molecular evolution of bacterial virulence factors. Nucleic Acids Res 40 (Database issue):D641-D645). The markers for the antibiotic resistance genes were based on all protein sequences (7828 protein sequences) from version 1.1 of the Antibiotic Resistance Genes Database (http://ardb.cbcb.umd.edu/ - Liu, B. and Pop., M., NAR, 2009, ARDB-Antibiotic Resistance Genes Database. Nucleic Acids Res 37(Database issue):D443-D447). Significance between groups was assessed by fitting a negative binomial regression model accounting for excess zero-count data if needed.

### Detection of pathogens by Luminex in stool samples

Stool samples were subjected to a series of mechanical disruption steps (3 x 60 s) using a FastPrep apparatus and Lysing Matrix B tubes (MP Biochemicals), the simultaneous extraction of DNA and RNA was performed with the QIAamp MinElute Virus Spin Kit (QIAGEN). Nucleic acids were detected using the Gastrointestinal Pathogen Panel (xTAG GPP) of sequence-specific primers with the Luminex 200 System according to recommendations by the manufacturer (Luminex Molecular Diagnostics, Inc., Toronto, Canada). The analytes detected were adenovirus serotypes 40/41, Campylobacter (C. jejuni, C. coli, and C. lari), Clostridium difficile toxins A/B, Cryptosporidium (C. parvum and C. hominis), Entamoeba histolytica, Escherichia coli 0157, Enterotoxigenic E. coli (ETEC) toxins LT/ST, Giardia (G. lamblia also knows as G. intestinalis and G. duodenalis), Norovirus GI/GII, Rotavirus A, Shiga-like toxin producing E. coli (STEC) stx1/stx2, Vibrio cholera, and Yersinia enterocolitica. The results of the detection of Salmonella and Shigella were not considered due to inconsistencies in the control samples.

### Stool metabolite analysis

To gain knowledge beyond compositional aspect of the stool microbiota, the inventors explored the biochemical composition of the stools using a well-established metabonomics approach based on proton Nuclear Magnetic Resonance Spectroscopy (1H NMR). 1H NMR Metabonomics of stools allows the quantitative profiling of major metabolites, including amino acids, major organics acids (lactate, succinate, citrate, etc.) and carbohydrates, and therefore open a unique window to monitor gut metabolic functionality.

Metabolic profiling of stool samples was adapted from our previously published method (Martin et al., 2014, Impact of breast-feeding and high- and low-protein formula on the metabolism and growth of infants from overweight and obese mothers. Pediatr Res 75, 535-543. doi: 10.1038/pr.2013.250). Briefly, 80-100mg offrozen stool was sampled from the stool collection tube, weighed and freeze dried. Dried samples were suspended in 1.2 mL of deuterated phosphate buffer solution 0.2 M KH2PO4, containing 0.3mM of sodium azide as anti-bacterial agent and 1 mM of sodium 3-(trimethylsilyl)-[2,2,3,3-2H4]-1-propionate as NMR chemical shift reference. The homogenates were centrifuged at 17,000 x g for 10 minutes and 5500 µL of the supernatant were transferred into 5 mm NMR tubes. 1H NMR metabolic profiles were acquired with a Bruker Avance III 600 MHz spectrometer equipped with a 5 mm cryoprobe at 300K (Bruker, Biospin, Germany) using a standard pulse sequence and a spin-echo pulse sequence with water suppression and processed using TOPSPIN (vs 3.2., Bruker) software package. Data processing and analysis was conducted as previously reported (Martin et al., 2014). Influential metabolites identified from the multivariate data analysis were relatively quantified by signal integration and analysed using Kruskal-Wallis tests. Due to the exploratory nature of the study p-values were not corrected for multiple testing.

### Results

### Stool microbiota composition based on 16S rDNA analysis

The global average profile of the three groups at genus level has revealed that although the global microbial composition of the Control and Test groups showed a similar formula-fed pattern, the Test group tends to be more similar to the BF group than the Control group. See figure 1 that provides the general profile for the 3 groups.

Statistical analyses identified several taxa differentially present between the Control and Test groups. Indeed, when the differences of microbiota composition at genus level was statistically analysed (Wilcoxon rank test, no correction for multiple testing), the Control and Test groups were different by six taxa (see table 4). Three taxa had median values of zero in all groups, showing counts for few outliers only. The others are shown in Figures 2. The significance of these three taxa (*Bifidobacterium, Escherichia and Peptostreptococacceae_uncl*) as main discriminants between Control and Test groups was confirmed by random forest analysis (mean decrease accuracy of 0.013 to 0.006). There is especially an up-regulation of the population of *Bifidobacteria* and a down regulation of the populations of *Escherichia* and/or *Peptostreptococcaceae,* in comparison to the global microbiota of the Control group.

**Table 4: Wilcoxon rank test at genus level was used to evaluate significant differences between Control and Test group indicated by the p-value. The BF group was not used for the statistical tests but values are shown as references. Values for Test, Control and BF are medians of the relative abundance of the indicated genus. The FDR q-are p-values that correct for multiple testing at a defined false discovery rate.**

| Genus | p-value | FDR q-value | Test | Control | BF (reference) |
|---|---|---|---|---|---|
| *Escherichia* | 0.008207 | 0.15888 | 1.616 | 4.432 | 2.196 |
| *Bifidobacterium* | 0.010146 | 0.15888 | 82.678 | 74.433 | 90.684 |
| *Coprobacillaceae_g__uncl* | 0.010592 | 0.15888 | 0 | 0 | 0 |
| *Peptostreptococcaceae_g__uncl* | 0.0258 | 0.2608 | 0.172 | 0.313 | 0 |
| *Dorea* | 0.033119 | 0.2608 | 0 | 0 | 0 |
| *Megamonas* | 0.035533 | 0.2608 | 0 | 0 | 0 |

The alpha diversity of each sample was calculated using a metric which takes into account the phylogenetic distances between the OTUs and their distribution in the three compared groups, see Figure 3. Although the diversity of the BF group is significantly lower than the diversity of both formula groups, the diversity of the Test group is significantly reduced (the mean is reduced by 0.19 units) compared to the Control group and is therefore closer to the BF group.

The difference of the global microbiota composition from the 16S rDNA data of the three groups was assessed by ordination (see Figure 4). Statistics based on random permutations of the redundancy analysis (RDA) showed that the three groups can significantly be separated at genus level (p<0.001). The centroids of the BF and Control groups were clearly separated, whereas the Test group was in an intermediate position between the BF and Control group.

### Pathogen load in stool samples

A subset of stool samples was available for analysis of specific pathogen load by the Luminex xTAG Gastrointestinal Pathogen Panel. Table 5 depicts the number of infants with at least one pathogen detected in the stool collected at 3 months of age. The number of infants with a detectable viral pathogen was very similar between the Test and Control group accounting for 28% and 31.5% respectively. Most frequently detected was Norovirus. On the other hand, 14% of infants fed the Test formula had detectable bacterial pathogens in the stool while 26% of the stool from Control infant showed at least one bacterial pathogen. However, this difference did not reach statistical significance (Odds ratio 0.46, p=0.15). By far Clostridium difficile, based on toxin A/B, was the most frequently detected pathogen in these European infants. Eukaryotic (Protista) pathogens were only very rarely detected with 2% of Test formula fed infants and 5.6% of Control formula fed infants showing Cryptosporidium in stool.

**Table 5: Number of infants with presence of at least one pathogen in stool at 3 months of age. Odds ratio and two tailed p-value by Fisher Exact Probability Test were calculated.**

| | **Test** | **Control** | | |
|---|---|---|---|---|
| **Pathogens** | n % | n % | Odds ratio | *p-value* |
| | | | (95% CI) | |
| **Viral** | 14/50 | 17/54 | 0.846 | *0.83* |
| | ***28%*** | ***31.5%*** | (0.364 -1.967) | |
| Adenovirus 40/41 | 4/50 | 0/54 | | |
| *N*orovirus GI/GII | 10/50 | 15/54 | | |
| Rotavirus A | 1/50 | 2/54 | | |
| **Bacterial** | 7/50 | 14/54 | 0.465 | *0.149* |
| | ***14%*** | ***26%*** | (0.170 -1.270) | |
| C. difficile toxin A/B | 7/50 | 12/54 | | |
| Campylobacter | 0/50 | 1/54 | | |
| E. coli O 157 | 0/50 | 1/54 | | |
| ETEC LT/ST | 0/50 | 0/54 | | |
| STEC stx1/stx2 | 0/50 | 0/54 | | |
| Vibrio cholerae | 0/50 | 0/54 | | |
| Yersinia enterocolitia | 0/50 | 0/54 | | |
| **Protist** | 1/50 | 3/54 | 0.347 | *0.619* |
| | ***2%*** | ***5.6%*** | (0.035 - 3.45) | |
| Cryptosporidium | 1/50 | 3/54 | | |
| Entamoeba histolytica | 0/50 | 0/54 | | |
| Giardia | 0/50 | 0/54 | | |

Pathogen load in the infant stool samples was also evaluated using the metagenome dataset. For Clostridium difficile 36% of infants in the Test and 46% in the Control group were carriers according to the metagenome data. A similar pattern was observed for C. difficile toxin A/B by the Luminex PCR method with 14% of infants in the Test and 22% of infants in the Control had detectable levels.

### Selected first functional aspects of stool microbiota

Besides looking at pathogens, the inventors also queried the metagenome dataset for the presence of genes encoding known virulence factors or antibiotic resistance genes. Figure 5 summarizes the results for known virulence genes with and without multiple testing corrections. In total the inventors detected 7 genes encoding known virulence factors whose levels appeared significantly different between the Control and the Test groups. Of those 7 genes, 5 appeared different between Control and Test, as well as between Control and BF, but not between Test and BF, indicating that the Test group was closer to the BF reference for those genes. The 2 further differential genes appeared different between all 3 groups.

With respect to genes encoding antibiotic resistance genes, the inventors detected a total of 8 genes encoding known antibiotic resistance genes whose levels appeared significantly different between the Control and the Test groups, see figure 6. Of those 8 genes, 4 appeared different between Control and Test, as well as between Control and BF, but not between Test and BF, indicating that the Test group was closer to the BF reference for those genes. The 4 other genes appeared only different between the Test and Control groups, but not between either of the formula groups and the BF reference group.

### Stool metabolic signature

Multivariate data analysis identified influential metabolites that discriminate between the Test and Control groups and the breast-fed reference group (see Figures 7). The content of the stools in some amino acids and organic acids was relatively different between the test and control formula, the differences observed in the test formula varying towards the values observed on the stool of breast-fed infants. Namely, phenylalanine and isoleucine levels were different between Test and Control fed infants and different from the breast-fed infant stools. Tyrosine were not significantly different between Test and Control, but different from the breast-fed reference. On the other hand lactate levels were higher in the stool of Test formula fed infants as compared to Control, while the breast-fed reference samples did not reach a statistical significant difference to the formula fed infants.

### Conclusion

All these different analyses show that the global microbial composition of Test group (i.e. infants fed an infant formula according to the invention) tends to be more similar to the BF group (i.e. infants fed exclusively with human breast milk) than the Control group (i.e. infants fed a conventional nutritional composition).

Indeed, this randomized placebo controlled two-center clinical trial showed that supplementing a standard starter infant formula with the 2 specific human milk oligosaccharides 2'FL and LNnT modulates the gut microbiota at 3 months of age, as assessed from stool samples. Notably, global microbiota composition and functional measures of the Test formula fed infants were not only different from the Controls, but got closer to the breast-fed reference. Specifically, this intermediate position of the Test group stool microbiota between the Control and the breast-fed reference groups is seen in the alpha-diversity plot, redundancy analysis at genus level and also the relative abundance comparison of specific taxa at the genus level. Major contributors to the observed shift of the Test group to the BF reference group are bacteria from the taxa *Bifidobacterium, Escherichia* and *Peptostreptococcaceae.* This shift in the microbiota composition is further corroborated by the observed change in the stool metabolic content of gut bacteria metabolites derived from milk digestion as seen by 1H-NMR metabonomics. This indicates that the HMOs 2'FL and LNnT in the Test formula not only affect the composition, but also the gut microbial function.

To further highlight a health related advantage for the infant, the inventors have investigated also the presence of specific bona fide pathogens and virulence factors at large. Although not reaching statistical significance, the bacterial pathogen C. difficile showed apparent lower levels, both by toxin A/B specific PCR amplification and by metagenome analysis, in the test group compared to the Control and approaching the lower levels observed in the BF reference in the metagenome data. Noteworthy, several known virulence and antibiotic resistance genes detected by metagenome analysis in the Test group appeared different in abundance when compared to Control group infant stool and similar to the abundance in the BF reference. Together and in the assumption the BF reference is the standard, these observations indicate that the gut host microbial ecology in the Test group might be less favorable to allow putative harmful bacteria.

The higher levels of the faecal free amino acids phenylalanine, tyrosine and isoleucine in the formula groups compared to the BF reference may either relate to increased proteolytic activity or excess of amino acids enriched in the formula but not absorbed in the upper gut.

The present findings with the Test formula shows that the addition of the HMOs 2'FL and LNnT to a formula tend to reduce the content in free amino acid in the stools, whilst stimulating the production of lactate. These changes describe the potential of 2'FL and LNnT to induce gut microbial metabolic changes towards the levels of metabolites seen in the stool of BF infants, and therefore towards inducing a metabolic equivalence with the breast milk.

Together the stool microbiota and metabolic signature show that the addition of 2 individual, structurally very specific HMOs to a starter infant formula shift the gut microbiota, evaluated in stool, both in global composition and function towards that observed in BF infants. Globally, the Test group infants position between the Control formula infants and the BF infants. Yet, for some specific measures the Test group even appeared identical to the BF reference group.

A nutritional composition comprising at least one fucosylated oligosaccharide and at least one N-acetylated oligosaccharide, such as 2FL and LNnT, appears to be very efficient in infants or young children in promoting and/or inducing in said infants or young children a global microbiota in the gut that is closer to the global microbiota in the gut of infants or young children fed exclusively with human breast milk, in comparison to the global microbiota in the gut of infants or young children fed predominantly or exclusively with a conventional nutritional composition not comprising said oligosaccharides.

It is also therefore thought to be particularly efficient for use in providing a healthy growth, for use in providing a healthy immune system, for use in providing a healthy gut function and/or for use in preventing and/or treating gut microbiota dysbiosis in infants or young children.

## Claims

1. Nutritional composition comprising at least one fucosylated oligosaccharide and at least one N-acetylated oligosaccharide for use in promoting and/or inducing in infants or young children a global microbiota in the gut that is closer to the global microbiota in the gut of infants or young children fed exclusively with human breast milk, in comparison to the global microbiota in the gut of infants or young children fed predominantly or exclusively with a conventional nutritional composition not comprising said oligosaccharides.

2. Nutritional composition for use according to claim 1 wherein the fucosylated oligosaccharide is selected from the list consisting of 2'-fucosyllactose, 3'fucosyllactose, difucosyllactose, lacto-N-fucopentaose I, lacto-N-fucopentaose II, lacto-N-fucopentaose III, lacto-N-fucopentaose V, lacto-N-fucohexaose, lacto-N-difucohexaose I, fucosyllacto-N-hexaose, fucosyllacto-N-neohexaose I, fucosyllacto-N-neohexaose II, difucosyllacto-N-hexaose I, difucosyllacto-N-neohexaose I, difucosyllacto-N-neohexaose II, fucosyl-para-Lacto-N-hexaose, and any combination thereof.

3. Nutritional composition for use according to any one of the preceding claims, wherein the fucosylated oligosaccharide comprises a 2' fucosyl-epitope.

4. Nutritional composition for use according to any one of the preceding claims, wherein the N-acetylated oligosaccharide is lacto-N-tetraose (LNT), lacto-N-neotetraose (LNnT) or any combination thereof.

5. Nutritional composition for use according to any one of the preceding claims, wherein the N-acetylated oligosaccharide is lacto-N-neotetraose (LNnT), para-lacto-N-neohexaose (para-LNnH) or any combination thereof, preferably wherein the N-acetylated oligosaccharide is lacto-N-neotetraose (LNnT).

6. Nutritional composition for use according to any one of the preceding claims, comprising 2'-fucosyllactose and lacto-N-neotetraose (LNnT), or comprising an oligosaccharide mixture consisting of 2'-fucosyllactose (2'FL) and lacto-N-neotetraose (LNnT).

7. Nutritional composition for use according to any one of the preceding claims, wherein the fucosylated oligosaccharide(s) is/are in a total amount of 0.8-1.5 g/L of the composition and/or in a total amount of 0.62-1.16 g/100g of composition on a dry weight basis; and/or
the N-acetylated oligosaccharide(s) is/are in a total amount of 0.5-0.8 g/L of the composition and/or in a total amount of 0.39-0.62 g/100g of composition on a dry weight basis.

8. Nutritional composition for use according to any one of the preceding claims, comprising at least another oligosaccharide(s) and/or fiber(s) and/or precursor(s) thereof selected from the list comprising GOS, FOS, XOS, inulin, polydextrose, sialylated oligosaccharides, sialic acid, fucose and any combination thereof.

9. Nutritional composition for use according to any one of the preceding claims, wherein said nutritional composition is an infant formula, a starter infant formula, a follow-on or follow-up infant formula, a preterm formula, a baby food, an infant cereal composition, a fortifier or a supplement, said infant preferably being under 6 months of age.

10. Nutritional composition for use according to any one of the preceding claims, wherein said infants or young children have a fragile or unbalanced microbiota or dysbiosis of microbiota, such as preterm infants, infants born by Caesarean-section, infants born small for gestational age or with low birth weight, hospitalized infants/young children, infants/young children treated or having been treated by antibiotics and/or infants/young children suffering or having suffered from gut infection and/or gut inflammation.

11. Nutritional composition for use according to any one of the preceding claims, wherein the global microbiota in the gut refers to the composition and/or the function of the entire microbiota in the gut, such as the relative taxonomic abundance, the diversity, the activity and/or the functionality of said microbiota.

12. Nutritional composition for use according to any one of the preceding claims, wherein said promotion and/or induction involves an up-regulation of the population of *Bifidobacterium* and/or a down regulation of the populations of *Escherichia* and/or *Peptostreptococcaceae,* in comparison to the global microbiota in the gut of infants or young children fed predominantly or exclusively with the conventional nutritional composition not comprising said oligosaccharides.

13. Nutritional composition for use according to any one of the preceding claims, wherein said promotion and/or induction involves a reduction of pathogen(s), especially the amount of the bacterial pathogen *Clostridium difficile,* and/or a reduction of virulence factor(s), in comparison to the global microbiota in the gut of infants or young children fed predominantly or exclusively with the conventional nutritional composition not comprising said oligosaccharides.

14. Nutritional composition for use according to any one of the preceding claims, wherein said promotion and/or induction involves a reduction of the production of free amino acids and/or a stimulation of the production of lactate, in comparison to the global microbiota in the gut of infants or young children fed predominantly or exclusively with the conventional nutritional composition not comprising said oligosaccharides.

15. Nutritional composition for use according to any one of the preceding claims, wherein said promoted and/or inducted global microbiota in the gut of infants and/or young children has an alpha diversity significantly reduced in comparison to the global microbiota in the gut of infants or young children fed predominantly or exclusively with the conventional nutritional composition not comprising the oligosaccharides.

16. Nutritional composition for use according to any one of the preceding claims, wherein said promoted and/or inducted global microbiota in the gut of infants and/or young children has an alpha diversity reduced of at least 0.10 units, such as at least 0.12 units or at least 0.15 units, for example 0.19 units, in comparison to the global microbiota in the gut of infants or young children fed predominantly or exclusively with the conventional nutritional composition not comprising the oligosaccharides.

17. Nutritional composition for use according to any one of the preceding claims, wherein said promotion and/or induction of the global microbiota in the gut is measurable in the stools of said infants or young children, for example at or after 1, 4, 6 or 8 weeks of age, and for example after 1, 4, 6 or 8 weeks of feeding with said nutritional composition.

18. Nutritional composition for use according to any one of the preceding claims, wherein said nutritional composition is fed or intended to be fed during the first 1, 2 4, 8, or 12 weeks of life, or during the first 2, 4, 6, 8 or 12 months of life.

## Patentansprüche

1. Nährstoffzusammensetzung, umfassend mindestens ein fucosyliertes Oligosaccharid und mindestens ein N-acetyliertes Oligosaccharid zur Verwendung beim Fördern und/oder Induzieren einer globalen Mikrobenflora im Darm bei Säuglingen oder Kleinkindern, die im Vergleich mit der globalen Mikrobenflora im Darm von Säuglingen oder Kleinkindern, die vorwiegend oder ausschließlich mit einer herkömmlichen Nährstoffzusammensetzung, die diese Oligosaccharide nicht umfasst, ernährt wurden, näher an der globalen Mikrobenflora im Darm von Säuglingen oder Kleinkindern ist, die ausschließlich mit menschlicher Muttermilch ernährt wurden.

2. Nährstoffzusammensetzung zur Verwendung nach Anspruch 1, wobei das fucosylierte Oligosaccharid ausgewählt ist aus der Liste, bestehend aus 2'-Fucosyllactose, 3'-Fucosyllactose, Difucosyllactose, Lacto-N-fucopentaose I, Lacto-N-fucopentaose II, Lacto-N-fucopentaose III, Lacto-N-fucopentaose V, Lacto-N-fucohexaose, Lacto-N-difucohexaose I, Fucosyllacto-N-hexaose, Fucosyllacto-N-neohexaose I, Fucosyllacto-N-neohexaose II, Difucosyllacto-N-hexaose I, Difucosyllacto-N-neohexaose I, Difucosyllacto-N-neohexaose II, Fucosyl-para-lacto-N-hexaose und einer beliebigen Kombination davon.

3. Nährstoffzusammensetzung zur Verwendung nach einem der vorstehenden Ansprüche, wobei das fucosylierte Oligosaccharid ein 2'-Fucosyl-Epitop umfasst.

4. Nährstoffzusammensetzung zur Verwendung nach einem der vorstehenden Ansprüche, wobei das N-acetylierte Oligosaccharid Lacto-N-tetraose (LNT), Lacto-N-neotetraose (LNnT) oder eine beliebige Kombination davon ist.

5. Nährstoffzusammensetzung zur Verwendung nach einem der vorstehenden Ansprüche, wobei das N-acetylierte Oligosaccharid Lacto-N-neotetraose (LNnT), para-Lacto-N-neohexaose (para-LNnH) oder eine beliebige Kombination davon ist, wobei vorzugsweise das N-acetylierte Oligosaccharid Lacto-N-neotetraose (LNnT) ist.

6. Nährstoffzusammensetzung zur Verwendung nach einem der vorstehenden Ansprüche, umfassend 2'-Fucosyllactose und Lacto-N-neotetraose (LNnT) oder umfassend ein Oligosaccharidgemisch, bestehend aus 2'-Fucosyllactose (2'FL) und Lacto-N-neotetraose (LNnT).

7. Nährstoffzusammensetzung zur Verwendung nach einem der vorstehenden Ansprüche, wobei das/die fucosylierte(n) Oligosaccharid(e) in einer Gesamtmenge von 0,8 - 1,5 g/l der Zusammensetzung und/oder in einer Gesamtmenge von 0,62 - 1,16 g/100 g der Zusammensetzung auf einer Trockengewichtsbasis vorliegt/vorliegen; und/oder wobei das/die N-acetylierte(n) Oligosaccharid(e) in einer Gesamtmenge von 0,5 - 0,8 g/l der Zusammensetzung und/oder in einer Gesamtmenge von 0,39 - 0,62 g/100 g der Zusammensetzung auf einer Trockengewichtsbasis vorliegt/vorliegen.

8. Nährstoffzusammensetzung zur Verwendung nach einem der vorstehenden Ansprüche, umfassend mindestens ein anderes / andere Oligosaccharid(e) und/oder (eine) Faser(n) und/oder (einen) Vorläufer davon, ausgewählt aus der Liste, umfassend GOS, FOS, XOS, Inulin, Polydextrose, sialylierte Oligosaccharide, Sialinsäure, Fucose und beliebige Kombination davon.

9. Nährstoffzusammensetzung zur Verwendung nach einem der vorstehenden Ansprüche, wobei die Nährstoffzusammensetzung eine Säuglingsformel, eine Säuglingsanfangsformel, eine Säuglings-Nachfolgeformel, eine Frühgeborenenformel, eine Babynahrung, eine Getreidezusammensetzung für Säuglinge, ein Stärkungsmittel oder ein Ergänzungsmittel ist, wobei der Säugling vorzugsweise unter 6 Monate alt ist.

10. Nährstoffzusammensetzung zur Verwendung nach einem der vorstehenden Ansprüche, wobei die Säuglinge oder Kleinkinder eine empfindliche oder unausgeglichene Mikrobenflora oder eine Dysbiose der Mikrobenflora aufweisen, wie frühgeborene Säuglinge, per Kaiserschnitt geborene Säuglinge, für das Gestationsalter zu klein oder mit niedrigem Geburtsgewicht geborene Säuglinge, hospitalisierte Säuglinge/Kleinkinder, Säuglinge/Kleinkinder, die mit Antibiotika behandelt werden oder wurden und/oder Säuglinge/Kleinkinder, die an einer Darminfektion und/oder Darmentzündung leiden oder gelitten haben.

11. Nährstoffzusammensetzung zur Verwendung nach einem der vorstehenden Ansprüche, wobei sich die globale Mikrobenflora im Darm auf die Zusammensetzung und/oder die Funktion der gesamten Mikrobenflora im Darm bezieht, wie die relative taxonomische Häufigkeit, die Diversität, die Aktivität und/oder die Funktionalität der Mikrobenflora.

12. Nährstoffzusammensetzung zur Verwendung nach einem der vorstehenden Ansprüche, wobei die Förderung und/oder Induzierung ein Hochregulieren der Population von *Bifidobacterium* und/oder ein Herunterregulieren der Populationen von *Escherichia* und/oder *Peptostreptococcaceae* im Vergleich zur globalen Mikrobenflora im Darm von Säuglingen oder Kleinkindern involviert, die vorwiegend oder ausschließlich mit der herkömmlichen Nährstoffzusammensetzung, die keine Oligosaccharide umfasst, ernährt werden.

13. Nährstoffzusammensetzung zur Verwendung nach einem der vorstehenden Ansprüche, wobei die Förderung und/oder Induzierung eine Verringerung von Pathogen(en) involviert, insbesondere der Menge des bakteriellen Pathogens *Clostridium difficile,* und/oder eine Verringerung von Virulenzfaktor(en) im Vergleich zu der globalen Mikrobenflora im Darm von Säuglingen oder Kleinkindern, die vorwiegend oder ausschließlich mit der herkömmlichen Nährstoffzusammensetzung, die keine Oligosaccharide umfasst, ernährt werden.

14. Nährstoffzusammensetzung zur Verwendung nach einem der vorstehenden Ansprüche, wobei die Förderung und/oder Induzierung eine Verringerung der Produktion freier Aminosäuren und/oder eine Stimulation der Produktion von Lactat im Vergleich zu der globalen Mikrobenflora im Darm von Säuglingen oder Kleinkindern involviert, die vorwiegend oder ausschließlich mit der herkömmlichen Nährstoffzusammensetzung, die keine Oligosaccharide umfasst, ernährt werden.

15. Nährstoffzusammensetzung zur Verwendung nach einem der vorstehenden Ansprüche, wobei die geförderte und/oder induzierte globale Mikrobenflora im Darm von Säuglingen und/oder Kleinkindern eine Alpha-Diversität aufweist, die im Vergleich zu der globalen Mikrobenflora im Darm von Säuglingen oder Kleinkindern, die vorwiegend oder ausschließlich mit der herkömmlichen Nährstoffzusammensetzung, die keine Oligosaccharide umfasst, ernährt werden, signifikant verringert ist.

16. Nährstoffzusammensetzung zur Verwendung nach einem der vorstehenden Ansprüche, wobei die geförderte und/oder induzierte globale Mikrobenflora im Darm von Säuglingen und/oder Kleinkindern eine Alpha-Diversität aufweist, die im Vergleich zu der globalen Mikrobenflora im Darm von Säuglingen oder Kleinkindern, die vorwiegend oder ausschließlich mit der herkömmlichen Nährstoffzusammensetzung, die keine Oligosaccharide umfasst, ernährt werden, um mindestens 0,10 Einheiten verringert ist, wie um mindestens 0,12 Einheiten oder um mindestens 0,15 Einheiten, zum Beispiel um 0,19 Einheiten.

17. Nährstoffzusammensetzung zur Verwendung nach einem der vorstehenden Ansprüche, wobei die Förderung und/oder Induzierung der globalen Mikrobenflora im Darm in den Stuhlgängen der Säuglinge oder Kleinkinder messbar ist, zum Beispiel im Alter von 1, 4, 6 oder 8 Wochen oder danach, und zum Beispiel nach 1, 4, 6 oder 8 Wochen des Ernährens mit der Nährstoffzusammensetzung.

18. Nährstoffzusammensetzung zur Verwendung nach einem der vorstehenden Ansprüche, wobei die Nährstoffzusammensetzung während der ersten 1, 2, 4, 8 oder 12 Lebenswochen oder während der ersten 2, 4, 6, 8 oder 12 Lebensmonate gefüttert wird oder gefüttert werden soll.

## Revendications

1. Composition nutritionnelle comprenant au moins un oligosaccharide fucosylé et au moins un oligosaccharide N-acétylé utilisable pour promouvoir et/ou induire chez des nourrissons ou jeunes enfants un microbiote global dans l'intestin qui est plus proche du microbiote global dans l'intestin de nourrissons ou jeunes enfants alimentés exclusivement par du lait maternel humain, par comparaison avec le microbiote global dans l'intestin de nourrissons ou jeunes enfants alimentés principalement ou exclusivement par une composition nutritionnelle classique ne comprenant pas lesdits oligosaccharides.

2. Composition nutritionnelle pour utilisation selon la revendication 1 dans laquelle l'oligosaccharide fucosylé est choisi dans la liste constituée de 2'-fucosyllactose, 3'-fucosyllactose, difucosyllactose, lacto-N-fucopentaose I, lacto-N-fucopentaose II, lacto-N-fucopentaose III, lacto-N-fucopentaose V, lacto-N-fucohexaose, lacto-N-difucohexaose I, fucosyllacto-N-hexaose, fucosyllacto-N-néohexaose I, fucosyllacto-N-néohexaose II, difucosyllacto-N-hexaose I, difucosyllacto-N-néohexaose I, difucosyllacto-N-néohexaose II, fucosyl-para-lacto-N-hexaose, et n'importe quelle combinaison de ceux-ci.

3. Composition nutritionnelle pour utilisation selon l'une quelconque des revendications précédentes, dans laquelle l'oligosaccharide fucosylé comprend un épitope de 2'-fucosyle.

4. Composition nutritionnelle pour utilisation selon l'une quelconque des revendications précédentes, dans laquelle l'oligosaccharide N-acétylé
est le lacto-N-tétraose (LNT), le lacto-N-néotétraose (LNnT) ou n'importe quelle combinaison de ceux-ci.

5. Composition nutritionnelle pour utilisation selon l'une quelconque des revendications précédentes, dans laquelle l'oligosaccharide N-acétylé est le lacto-N-néotétraose (LNnT), le para-lacto-N-néohexaose (para-LNnH) ou n'importe quelle combinaison de ceux-ci, de préférence dans laquelle l'oligosaccharide N-acétylé est le lacto-N-néotétraose (LNnT).

6. Composition nutritionnelle pour utilisation selon l'une quelconque des revendications précédentes, comprenant du 2'-fucosyllactose et du lacto-N-néotétraose (LNnT), ou comprenant un mélange d'oligosaccharides constitué de 2'-fucosyllactose (2'FL) et de lacto-N-néotétraose (LNnT).

7. Composition nutritionnelle pour utilisation selon l'une quelconque des revendications précédentes, dans laquelle le(s) oligosaccharide(s) fucosylé(s) est/sont en une quantité totale de 0,8 à 1,5 g/L de la composition et/ou en une quantité totale de 0,62 à 1,16 g/100 g de composition sur une base de poids sec ; et/ou le(s) oligosaccharide(s) fucosylé(s) est/sont en une quantité totale de 0,5 à 0,8 g/L de la composition et/ou en une quantité totale de 0,39 à 0,62 g/100 g de composition sur une base de poids sec.

8. Composition nutritionnelle pour utilisation selon l'une quelconque des revendications précédentes, comprenant au moins un/des autre(s) oligosaccharide(s) et/ou fibre(s) et/ou précurseur(s) de ceux-ci choisi(s) parmi la liste comprenant GOS, FOS, XOS, inuline, polydextrose, oligosaccharides sialylés, acide sialique, fucose et n'importe quelle combinaison de ceux-ci.

9. Composition nutritionnelle pour utilisation selon l'une quelconque des revendications précédentes, dans laquelle ladite composition nutritionnelle est une préparation pour nourrissons, une préparation de première étape pour nourrissons, une préparation de suite ou de suivi pour nourrissons, une préparation pour prématurés, une alimentation pour bébés, une composition de céréale pour nourrissons, un fortifiant ou un complément, ledit nourrisson étant de préférence âgé de moins de 6 mois.

10. Composition nutritionnelle pour utilisation selon l'une quelconque des revendications précédentes, dans laquelle lesdits nourrissons ou jeunes enfants ont un microbiote fragile ou déséquilibré ou une dysbiose du microbiote, tels que des nourrissons prématurés, des nourrissons nés par césarienne, des nourrissons nés petits pour l'âge gestationnel ou présentant un faible poids à la naissance, des nourrissons/jeunes enfants hospitalisés, des nourrissons/jeunes enfants traités ou ayant été traités par des antibiotiques et/ou des nourrissons/jeunes enfants souffrant ou ayant souffert d'une infection intestinale ou d'une inflammation intestinale.

11. Composition nutritionnelle pour utilisation selon l'une quelconque des revendications précédentes, dans laquelle le microbiote global dans l'intestin désigne la composition et/ou la fonction du microbiote entier dans l'intestin, telle que l'abondance taxonomique relative, la diversité, l'activité et/ou la fonctionnalité dudit microbiote.

12. Composition nutritionnelle pour utilisation selon l'une quelconque des revendications précédentes, dans laquelle lesdites promotion et/ou induction impliquent une régulation à la hausse de la population de *Bifidobacterium* et/ou une régulation à la baisse des populations *d'Escherichia* et/ou de *Peptostreptococcaceae,* par comparaison avec le microbiote global dans l'intestin de nourrissons ou jeunes enfants alimentés principalement ou exclusivement avec la composition nutritionnelle classique ne comprenant pas lesdits oligosaccharides.

13. Composition nutritionnelle pour utilisation selon l'une quelconque des revendications précédentes, dans laquelle lesdites promotion et/ou induction impliquent une réduction d'agent(s) pathogène(s), spécialement la quantité de l'agent pathogène bactérien *Clostridium difficile,* et/ou une réduction de facteur(s) de virulence, par comparaison avec le microbiote global dans l'intestin de nourrissons ou jeunes enfants alimentés principalement ou exclusivement avec la composition nutritionnelle classique ne comprenant pas lesdits oligosaccharides.

14. Composition nutritionnelle pour utilisation selon l'une quelconque des revendications précédentes, dans laquelle lesdites promotion et/ou induction impliquent une réduction de la production d'acides aminés libres et/ou une stimulation de la production de lactate, par comparaison avec le microbiote global dans l'intestin de nourrissons ou jeunes enfants alimentés principalement ou exclusivement avec la composition nutritionnelle classique ne comprenant pas lesdits oligosaccharides.

15. Composition nutritionnelle pour utilisation selon l'une quelconque des revendications précédentes, dans laquelle ledit microbiote global promu et/ou induit dans l'intestin de nourrissons et/ou jeunes enfants a une diversité alpha significativement réduite par comparaison avec le microbiote global dans l'intestin de nourrissons ou jeunes enfants alimentés principalement ou exclusivement avec la composition nutritionnelle classique ne comprenant pas lesdits oligosaccharides.

16. Composition nutritionnelle pour utilisation selon l'une quelconque des revendications précédentes, dans laquelle ledit microbiote global promu et/ou induit dans l'intestin de nourrissons et/ou jeunes enfants a une diversité alpha réduite d'au moins 0,10 unité, telle que d'au moins 0,12 unité ou d'au moins 0,15 unité, par exemple de 0,19 unité, par comparaison avec le microbiote global dans l'intestin de nourrissons ou jeunes enfants alimentés principalement ou exclusivement avec la composition nutritionnelle classique ne comprenant pas lesdits oligosaccharides.

17. Composition nutritionnelle pour utilisation selon l'une quelconque des revendications précédentes, dans laquelle lesdites promotion et/ou induction du microbiote global dans l'intestin sont mesurables dans les fèces desdits nourrissons ou jeunes enfants, par exemple à, ou après, un âge de 1, 4, 6 ou 8 semaines, et par exemple après 1, 4, 6 ou 8 semaines d'alimentation avec ladite composition nutritionnelle.

18. Composition nutritionnelle pour utilisation selon l'une quelconque des revendications précédentes, dans laquelle ladite composition nutritionnelle est administrée ou est prévue pour être administrée pendant les 1, 2 4, 8, ou 12 premières semaines de la vie, ou pendant les 2, 4, 6, 8 ou 12 premiers mois de la vie.
